# EUROPEAN PATENT APPLICATION

(11) **EP 3 591 043 A1**
(43) Date of publication of application: **08.01.2020**
(21) Application number: 19184883.7
(22) Date of filing: 08.07.2019
(51) Int. Cl.: C12N 9/02, C12N 15/01, C12P 7/64

(54) **A LONG-CHAIN DIBASIC ACID WITH LOW CONTENT OF FATTY ACID IMPURITY AND A METHOD OF PRODUCING THE SAME**

(30) Priority: 06.07.2018 CN 201810734188; 06.07.2018 CN 201810734323; 01.04.2019 CN 201910255480
(71) Applicant: Cathay Biotech Inc., Shanghai Pilot Free Trade Zone (CN); Cibt America Inc., Newark DE 19713 (US)
(72) Inventor: LIU, Wenbo, Shanghai, Shanghai 201203 (CN); XU, Min, Shanghai, Shanghai 201203 (CN); YANG, Chen, Shanghai, Shanghai 201203 (CN); CHOU, Howard, Shanghai, Shanghai 201203 (CN); LIU, Xiucai, Shanghai, Shanghai 201203 (CN)
(74) Representative: Vossius & Partner Patentanwälte Rechtsanwälte mbB

(57) **Abstract**

The invention relates to a long-chain dibasic acid with low content of fatty acid impurity and a method of producing it, in particular to the preparation of a long-chain dibasic acid producing strain by using directed evolution and homologous recombination, and to the fermentation production of a long-chain dibasic acid with low content of fatty acid impurity by using said strain. The invention relates to an isolated mutated CPR-b gene, homologous gene or variant thereof, relative to the GenBank Accession Number AY823228, taking the first base upstream of the start codon ATG as -1, comprising a base mutation -322G>A, and taking the first base downstream of the stop codon TAG as 1, comprising mutations 3'UTR.19C>T and 3'UTR.76_77insT. The invention also relates to a strain containing said mutated CPR-b gene, homologous gene or variant thereof, wherein, when the strain is used to produce a long-chain dibasic acid by fermentation, the content of fatty acid impurity in the fermentation product is significantly decreased.

SEQUENCE LISTING

<110>  Shanghai Cathay Biotech R&D Center Ltd.
       CIBT America Inc.

<120>  A Long-Chain Dibasic acid with Low Content of Fatty Acid Impurity
        and A Method of Producing the same

<130>  AC2281 EP

<150>  CN201810734188.0
<151>  2018-07-06

<150>  CN201810734323.1
<151>  2018-07-06

<150>  CN201910255480.9
<151>  2019-04-01

<160>  23

<170>  PatentIn version 3.5

<210>  1
<211>  19
<212>  DNA
<213>  Artificial Sequence

<220>
<223>  primer CPR-b-F

<400>  1
cgaagttgtt gggggatct                                                    19


<210>  2
<211>  20
<212>  DNA
<213>  Artificial Sequence

<220>
<223>  primer CPR-b-R

<400>  2
tatcccggca ttaccaacgg                                                   20


<210>  3
<211>  20
<212>  DNA
<213>  Artificial Sequence

<220>
<223>  primer CPR-b_Upstream-F

<400>  3
tttgcgcgag taacatgtgc                                                   20


<210>  4
<211>  20
<212>  DNA
<213>  Artificial Sequence

<220>
<223>  primer CPR-b_Upstream-R

<400>  4
   aatgattcct gcgaggggtg                                                    20


<210>  5
<211>  22
<212>  DNA
<213>  Artificial Sequence

<220>
<223>  primer CPR-b_Downstream-F

<400>  5
tttagtacag tatctccaat cc                                                22


<210>  6
<211>  20
<212>  DNA
<213>  Artificial Sequence

<220>
<223>  primer CPR-b_Downstream-R

<400>  6
acgtctatat tgtggatggc                                                   20


<210>  7
<211>  40
<212>  DNA
<213>  Artificial Sequence

<220>
<223>  primer CPR_HYG-F

<400>  7
ccgttggtaa tgccgggata gcatgcgaac ccgaaaatgg                             40


<210>  8
<211>  42
<212>  DNA
<213>  Artificial Sequence

<220>
<223>  primer CPR_HYG-R

<400>  8
ggattggaga tactgtacta aagctagcag ctggatttca ct                          42


<210>  9
<211>  1778
<212>  DNA
<213>  Artificial Sequence

<220>
<223>  HYG

<400> 9
ccgttggtaa tgccgggata gcatgcgaac ccgaaaatgg agcaatcttc cccggggcct 60

ccaaatacca actcacccga gagagataaa gagacaccac ccaccacgag acggagtata 120

tccaccaagg taagtaactc agagttaatg atacaggtgt acacagctcc ttccctagcc 180

attgagtggg tatcacatga cactggtagg ttacaaccac gtttagtagt tattttgtgc 240

aattccatgg ggatcaggaa gtttggtttg gtgggtgcgt ctactgattc ccctttgtct 300

ctgaaaatct tttccctagt ggaacacttt ggctgaatga tataaattca ccttgattcc 360

caccctccct tctttctctc tctctctgtt acacccaatt gaattttctt ttttttttta 420

ctttccctcc ttctttatca tcaaagataa gtaagtttat caattgccta ttcagaatga 480

aaaagcctga actcaccgcg acgtctgtcg agaagtttct catcgaaaag ttcgacagcg 540

tctccgacct catgcagctc tcggagggcg aagaatctcg tgctttcagc ttcgatgtag 600

gagggcgtgg atatgtcctc cgggtaaata gctgcgccga tggtttctac aaagatcgtt 660

atgtttatcg gcactttgca tcggccgcgc tcccgattcc ggaagtgctt gacattgggg 720

aattcagcga gagcctcacc tattgcatct cccgccgtgc acagggtgtc acgttgcaag 780

acctccctga aaccgaactc cccgctgttc tccagccggt cgcggaggcc atggatgcga 840

tcgctgcggc cgatcttagc cagacgagcg ggttcggccc attcggaccg caaggaatcg 900

gtcaatacac tacatggcgt gatttcatat gcgcgattgc tgatccccat gtgtatcact 960

ggcaaactgt gatggacgac accgtcagtg cgtccgtcgc gcaggctctc gatgagctca 1020

tgctttgggc cgaggactgc cccgaagtcc ggcacctcgt gcacgcggat ttcggctcca 1080

acaatgtcct cacggacaat ggccgcataa cagcggtcat tgactggagc gaggcgatgt 1140

tcggggattc ccaatacgag gtcgccaaca tcttcttctg gaggccgtgg ttggcttgta 1200

tggagcagca gacgcgctac ttcgagcgga ggcatccgga gcttgcagga tcgccgcggc 1260

tccgggcgta tatgctccgc attggtcttg accaactcta tcagagcttg gttgacggca 1320

atttcgatga tgcagcttgg gcgcagggtc gatgcgacgc aatcgtccga tccggagccg 1380

ggactgtcgg gcgtacacaa atcgcccgca gaagcgcggc cgtctggacc gatggctgtg 1440

tagaagtact cgccgatagt ggaaaccgac gccccagcac tcgtccgagg gcaaaggaat 1500

agtgtgctac ccacgcttac tccaccagag ctattaacat cagaaatatt tattctaata 1560

aataggatgc aaaaaaaaaa ccccccttaa taaaaaaaaa agaaacgatt ttttatctaa 1620

tgaagtctat gtatctaaca aatgtatgta tcaatgttta ttccgttaaa caaaaatcag 1680

tctgtaaaaa aggttctaaa taaatattct gtctagtgta cacattctcc caaaatagtg 1740

aaatccagct gctagcttta gtacagtatc tccaatcc 1778

<210>  10
<211>  21
<212>  DNA
<213>  Artificial Sequence

<220>
<223>  primer CPR-b-2F

<400>  10
attacgaaac ataggtcaac t                                                21


<210>  11
<211>  20
<212>  DNA
<213>  Artificial Sequence

<220>
<223>  primer CPR-b-2R

<400>  11
taaccatatc catacgtcgc                                                  20


<210>  12
<211>  330
<212>  DNA
<213>  Artificial Sequence

<220>
<223>  CPR-b-2

<400>  12
attacgaaac ataggtcaac tatatatact tgattaaatg ttatagaaac aataattatt      60

atctactcgt ctacttcttt ggcattggca ttggcattgg cattggcatt gccgttgccg     120

ttggtaatgc cgggatattt agtacagtat ctccaatccg gatttgagct attgtaaatc     180

agctgcaagt cattctccac cttcaaccag tacttatact tcatctttga cttcaagtcc     240

aagtcataaa tattacaagt tagcaagaac ttctggccat ccacaatata gacgttattc     300

acgttattat gcgacgtatg gatatggtta                                      330


<210>  13
<211>  2792
<212>  DNA
<213>  Artificial Sequence

<220>
<223>  mutated CPR-b gene

<400>  13
cgaagttgtt gggggatctc ctccgcagtt tatgttcatg tctttcccac tttggttgtg      60

attggggtag cgtagtgagt tggtgatttt cttttttcgc aggtgtctcc gatatcgaag     120

tttgatgaat ataggagcca gatcagcatg gtatattgcc tttgtagata gagatgttga     180

acaacaacta gctgaattac acaccaccgc taaacgatgc gcacagggtg tcaccgccaa     240

ctgacgttgg gtggagttgt tgttggcagg gccatattgc taaacgaaga gaagtagcac 300

aaaacccaag gttaagaaca attaaaaaaa ttcatacgac aattccacag ccatttacat 360

aatcaacagc gacaaatgag acagaaaaaa ctttcaacat ttcaaagttc cctttttcct 420

attacttctt tttttctttc cttcctttca tttcctttcc ttctgctttt attactttac 480

cagtcttttg cttgtttttg caattcctca tcctcctcct caccatggct ttagacaagt 540

tagatttgta tgtcatcata acattggtgg tcgctgtggc cgcctatttt gctaagaacc 600

agttccttga tcagccccag gacaccgggt tcctcaacac ggacagcgga agcaactcca 660

gagacgtctt gctgacattg aagaagaata ataaaaacac gttgttgttg tttgggtccc 720

agaccggtac ggcagaagat tacgccaaca aattgtcaag agaattgcac tccagatttg 780

gcttgaaaac catggttgca gatttcgctg attacgattg ggataacttc ggagatatca 840

ccgaagatat cttggtgttt ttcatcgttg ccacctacgg tgagggtgaa cctaccgaca 900

atgccgacga gttccacacc tggttgactg aagaagctga cactttgagt actttgagat 960

ataccgtgtt cgggttgggt aactccacct acgagttctt caatgctatt ggtagaaagt 1020

ttgacagatt gttgagtgag aaaggtggtg acagatttgc tgaatatgct gaaggtgacg 1080

acggcactgg caccttggac gaagatttca tggcctggaa ggataatgtc tttgacgcct 1140

tgaagaatga cttgaacttt gaagaaaagg aattgaagta cgaaccaaac gtgaaattga 1200

ctgagagaga tgacttgtct gctgccgact cccaagtttc cttgggtgag ccaaacaaga 1260

agtacatcaa ctccgagggc atcgacttga ccaagggtcc attcgaccac acccacccat 1320

acttggccag gatcaccgag accagagagt tgttcagctc caaggaaaga cactgtattc 1380

acgttgaatt tgacatttct gaatcgaact tgaaatacac caccggtgac catctagcca 1440

tctggccatc caactccgac gaaaacatca agcaatttgc caagtgtttc ggattggaag 1500

ataaactcga cactgttatt gaattgaagg cattggactc cacttacacc attccattcc 1560

caactccaat tacttacggt gctgtcatta gacaccattt agaaatctcc ggtccagtct 1620

cgagacaatt ctttttgtcg attgctgggt ttgctcctga tgaagaaaca aagaagactt 1680

tcaccagact tggtggtgac aaacaagaat tcgccaccaa ggttacccgc agaaagttca 1740

acattgccga tgccttgtta tattcctcca acaacactcc atggtccgat gttccttttg 1800

agttccttat tgaaaacatc caacacttga ctccacgtta ctactccatt tcttcttcgt 1860

cgttgagtga aaaacaactc atcaatgtta ctgcagtcgt tgaggccgaa gaagaagccg 1920

atggcagacc agtcactggt gttgttacca acttgttgaa gaacattgaa attgcgcaaa 1980

acaagactgg cgaaaagcca cttgttcact acgatttgag cggcccaaga ggcaagttca 2040

acaagttcaa gttgccagtg cacgtgagaa gatccaactt taagttgcca aagaactcca 2100

ccaccccagt tatcttgatt ggtccaggta ctggtgttgc cccattgaga ggtttcgtta 2160

gagaaagagt tcaacaagtc aagaatggtg tcaatgttgg caagactttg ttgttttatg 2220

gttgcagaaa ctccaacgag gactttttgt acaagcaaga atgggccgag tacgcttctg 2280

ttttgggtga aaactttgag atgttcaatg ccttctctag acaagaccca tccaagaagg 2340

tttacgtcca ggataagatt ttagaaaaca gccaacttgt gcacgaattg ttgaccgaag 2400

gtgccattat ctacgtctgt ggtgacgcca gtagaatggc cagagacgtc cagaccacga 2460

tctccaagat tgttgccaaa agcagagaaa tcagtgaaga caaggccgct gaattggtca 2520

agtcctggaa agtccaaaat agataccaag aagatgtttg gtagactcaa acgaatctct 2580

ctttctccca acgcatttat gaatattctc attgaagttt tacatatgtt ctatatttca 2640

tttttttttt attatattac gaaacatagg tcaactatat atacttgatt aaatgttata 2700

gaaacaataa ttattatcta ctcgtctact tctttggcat tggcattggc attggcattg 2760

gcattgccgt tgccgttggt aatgccggga ta 2792

<210> 14
<211> 264
<212> DNA
<213> Artificial Sequence

<220>
<223> CPR-b_Upstream

<400> 14
gggggatcaa aagcggaaga tttgtgttgc ttgtgggttt tttcctttat ttttcatatg 60

atttctttgc gcaagtaaca tgtgccaatt tagtttgtga ttagcgtgcc ccacaattgg 120

catcgtggac gggcgtgttt tgtcataccc caagtcttaa ctagctccac agtctcgacg 180

gtgtctcgac gatgtcttct tccacccctc ccatgaatca ttcaaagttg ttgggggatc 240

tccaccaagg gcaccggagt taat 264

<210> 15
<211> 226
<212> DNA
<213> Artificial Sequence

<220>
<223> CPR-b_Downstream

<400> 15
tcccattacc gttgccgttg gcaatgccgg gatatttagt acagtatctc caatccggat 60

ttgagctatt gtagatcagc tgcaagtcat tctccacctt caaccagtac ttatacttca 120

tctttgactt caagtccaag tcataaatat tacaagttag caagaacttc tggccatcca 180

cgatatagac gttattcacg ttattatgcg acgtatggat gtggtt 226

<210> 16

<211>  5873
<212>  DNA
<213>  Artificial Sequence

<220>
<223>  vector pCIB2

<400>  16
gcgcccaata cgcaaaccgc ctctccccgc gcgttggccg attcattaat gcagctggca      60

cgacaggttt cccgactgga aagcgggcag tgagcgcaac gcaattaatg tgagttagct     120

cactcattag gcaccccagg ctttacactt tatgcttccg gctcgtatgt tgtgtggaat     180

tgtgagcgga taacaatttc acacaggaaa cagctatgac catgattacg aattcggtct     240

agtatgattg tcaataatga tgggtcatcg tttcctgatt cgacgttccc tgtggtgtcg     300

ttaaatagcc tgtctgaaat ctcctccatg attgtgttgg tgtgtgttgt ttgactttcc     360

caattgctta catttttttc ttcaaggatt cgctccaaaa tagacagaaa ttatcgcgac     420

aagtcagacg aacgtcgcac gaggcgaacc aaattcttta gaagcatacg aaaactcact     480

ttatttccat tagaagtatt aaattaacaa atatataata tacaggatac aaagtaaaag     540

cacgcttaag caaccaaagc ggaagcggta gcggattcgt atttccagtt aggtggcaag     600

acagcgacgg ttctgtagta tctggccaat ctgtggattc tagattcaat caaaatcaat     660

ctgaacttgg agtccttgtc ctttctgttt ctttccaagt gctttctgac agagacagcc     720

ttcttgatca agtagtacaa gtcttctggg atttctggag ccaaaccgtt ggatttcaag     780

attctcaaga tcttgttacc agtgacaacc ttggcttggg aaacaccgtg agcatctctc     840

aagataacac caatttgaga tggagtcaaa ccctttctgg cgtacttgat gacttgttca     900

acaacttcgt cagaagacaa cttgaaccaa gatggagcgt ttcttgagta tggaagagcg     960

gaggaggaaa tacctttacc ctaaaataac aagagctaat gttagtaatt tgaaaaaaaa    1020

gacgttgagc acgcacaccc catccacccc acaggtgaaa cacatcaaac gtagcaagaa    1080

caatagttgg ccctcccgtc aagggggcag gtaattgtcc aagtacttta gaaaagtatg    1140

tttttaccca taagatgaac acacacaaac cagcaaaagt atcaccttct gcttttcttg    1200

gttgaggttc aaattatgtt tggcaataat gcagcgacaa tttcaagtac ctaaagcgta    1260

tatagtaaca attctaggtc tgtatagtcg accgtaggtg aatcgtttac tttaggcaag    1320

accttgtccc tgataaagcc aggttgtact ttctattcat tgagtgtcgt ggtggtggta    1380

gtggtggttg attgggctgt tgtggtagta gtagtggttg tgatttggaa catacagatg    1440

aatgcatacg acccatgatg actgatttgt ttctttattg agttgatggt aagaaagaga    1500

agaagaggag gtaaaaaggt ggtagagtga aaaatttttt tctcttaaaa gtgagagaga    1560

gaaagagaaa aatttcactg cgaaacaaat ggttggggac acgacttttt tcaggaattt    1620

ttactcgaag cgtatatgca ggaaagttgt tgttagggaa tatggagcca caagagagct    1680

gcgaattcga gctcggtacc cggggatcct ctagagtcga cctgcaggca tgcgaacccg 1740

aaaatggagc aatcttcccc ggggcctcca aataccaact cacccgagag agagaaagag 1800

acaccaccca ccacgagacg gagtatatcc accaaggtaa gtaactcagg gttaatgata 1860

caggtgtaca cagctccttc cctagccatt gagtgggtat cacatgacac tggtaggtta 1920

caaccacgtt tagtagttat tttgtgcaat tccatgggga tcaggaagtt tggtttggtg 1980

ggtgcgtcta ctgattcccc tttgtctctg aaaatctttt ccctagtgga acactttggc 2040

tgaatgatat aaattcacct tgattcccac cctcccttct ttctctctct ctctgttaca 2100

cccaattgaa ttttcttttt ttttttactt tccctccttc tttatcatca aagataagta 2160

agtttatcaa ttgcctattc agaatgaaaa agcctgaact caccgcgacg tctgtcgaga 2220

agtttctcat cgaaaagttc gacagcgtct ccgacctcat gcagctctcg gagggcgaag 2280

aatctcgtgc tttcagcttc gatgtaggag ggcgtggata tgtcctccgg gtaaatagct 2340

gcgccgatgg tttctacaaa gatcgttatg tttatcggca ctttgcatcg gccgcgctcc 2400

cgattccgga agtgcttgac attggggaat tcagcgagag cctcacctat tgcatctccc 2460

gccgtgcaca gggtgtcacg ttgcaagacc tccctgaaac cgaactcccc gctgttctcc 2520

agccggtcgc ggaggccatg gatgcgatcg ctgcggccga tcttagccag acgagcgggt 2580

tcggcccatt cggaccgcaa ggaatcggtc aatacactac atggcgtgat ttcatatgcg 2640

cgattgctga tccccatgtg tatcactggc aaactgtgat ggacgacacc gtcagtgcgt 2700

ccgtcgcgca ggctctcgat gagctcatgc tttgggccga ggactgcccc gaagtccggc 2760

acctcgtgca cgcggatttc ggctccaaca atgtcctcac ggacaatggc cgcataacag 2820

cggtcattga ctggagcgag gcgatgttcg gggattccca atacgaggtc gccaacatct 2880

tcttctggag gccgtggttg gcttgtatgg agcagcagac gcgctacttc gagcggaggc 2940

atccggagct tgcaggatcg ccgcggctcc gggcgtatat gctccgcatt ggtcttgacc 3000

aactctatca gagcttggtt gacggcaatt tcgatgatgc agcttgggcg cagggtcgat 3060

gcgacgcaat cgtccgatcc ggagccggga ctgtcgggcg tacacaaatc gcccgcagaa 3120

gcgcggccgt ctggaccgat ggctgtgtag aagtactcgc cgatagtgga aaccgacgcc 3180

ccagcactcg tccgagggca aaggaatagt gtgctaccca cgcttactcc accagagcta 3240

ttaacatcag aaatatttat tctaataaat aggatgcaaa aaaaaaaccc cccttaataa 3300

aaaaaaaaga aacgattttt tatctaatga agtctatgta tctaacaaat gtatgtatca 3360

atgtttattc cgttaaacaa aaatcagtct gtaaaaaagg ttctaaataa atattctgtc 3420

tagtgtacac attctcccaa aatagtgaaa tccagctgct agcgtgtaag cttggcactg 3480

gccgtcgttt tacaacgtcg tgactgggaa aaccctggcg ttacccaact taatcgcctt 3540

gcagcacatc cccctttcgc cagctggcgt aatagcgaag aggcccgcac cgatcgccct 3600

tcccaacagt tgcgcagcct gaatggcgaa tggcgcctga tgcggtattt tctccttacg 3660

catctgtgcg gtatttcaca ccgcatatgg tgcactctca gtacaatctg ctctgatgcc 3720

gcatagttaa gccagccccg acacccgcca acacccgctg acgcgccctg acgggcttgt 3780

ctgctcccgg catccgctta cagacaagct gtgaccgtct ccgggagctg catgtgtcag 3840

aggttttcac cgtcatcacc gaaacgcgcg agacgaaagg gcctcgtgat acgcctattt 3900

ttataggtta atgtcatgat aataatggtt tcttagacgt caggtggcac ttttcgggga 3960

aatgtgcgcg gaacccctat ttgtttattt ttctaaatac attcaaatat gtatccgctc 4020

atgagacaat aaccctgata aatgcttcaa taatattgaa aaaggaagag tatgagtatt 4080

caacatttcc gtgtcgccct tattcccttt tttgcggcat tttgccttcc tgtttttgct 4140

cacccagaaa cgctggtgaa agtaaaagat gctgaagatc agttgggtgc acgagtgggt 4200

tacatcgaac tggatctcaa cagcggtaag atccttgaga gttttcgccc cgaagaacgt 4260

tttccaatga tgagcacttt taaagttctg ctatgtggcg cggtattatc ccgtattgac 4320

gccgggcaag agcaactcgg tcgccgcata cactattctc agaatgactt ggttgagtac 4380

tcaccagtca cagaaaagca tcttacggat ggcatgacag taagagaatt atgcagtgct 4440

gccataacca tgagtgataa cactgcggcc aacttacttc tgacaacgat cggaggaccg 4500

aaggagctaa ccgctttttt gcacaacatg ggggatcatg taactcgcct tgatcgttgg 4560

gaaccggagc tgaatgaagc cataccaaac gacgagcgtg acaccacgat gcctgtagca 4620

atggcaacaa cgttgcgcaa actattaact ggcgaactac ttactctagc ttcccggcaa 4680

caattaatag actggatgga ggcggataaa gttgcaggac cacttctgcg ctcggccctt 4740

ccggctggct ggtttattgc tgataaatct ggagccggtg agcgtgggtc tcgcggtatc 4800

attgcagcac tggggccaga tggtaagccc tcccgtatcg tagttatcta cacgacgggg 4860

agtcaggcaa ctatggatga acgaaataga cagatcgctg agataggtgc ctcactgatt 4920

aagcattggt aactgtcaga ccaagtttac tcatatatac tttagattga tttaaaactt 4980

catttttaat ttaaaaggat ctaggtgaag atcctttttg ataatctcat gaccaaaatc 5040

ccttaacgtg agttttcgtt ccactgagcg tcagaccccg tagaaaagat caaaggatct 5100

tcttgagatc ctttttttct gcgcgtaatc tgctgcttgc aaacaaaaaa accaccgcta 5160

ccagcggtgg tttgtttgcc ggatcaagag ctaccaactc tttttccgaa ggtaactggc 5220

ttcagcagag cgcagatacc aaatactgtc cttctagtgt agccgtagtt aggccaccac 5280

ttcaagaact ctgtagcacc gcctacatac ctcgctctgc taatcctgtt accagtggct 5340

gctgccagtg gcgataagtc gtgtcttacc gggttggact caagacgata gttaccggat 5400

aaggcgcagc ggtcgggctg aacggggggt tcgtgcacac agcccagctt ggagcgaacg 5460

acctacaccg aactgagata cctacagcgt gagctatgag aaagcgccac gcttcccgaa 5520

gggagaaagg cggacaggta tccggtaagc ggcagggtcg gaacaggaga gcgcacgagg 5580

gagcttccag ggggaaacgc ctggtatctt tatagtcctg tcgggtttcg ccacctctga 5640

cttgagcgtc gatttttgtg atgctcgtca ggggggcgga gcctatggaa aaacgccagc 5700

aacgcggcct ttttacggtt cctggccttt tgctggcctt ttgctcacat gttctttcct 5760

gcgttatccc ctgattctgt ggataaccgt attaccgcct ttgagtgagc tgataccgct 5820

cgccgcagcc gaacgaccga gcgcagcgag tcagtgagcg aggaagcgga aga 5873

<210> 17
<211> 20
<212> DNA
<213> Artificial Sequence

<220>
<223> primer HYG-F

<400> 17
ctcggagggc gaagaatctc 20

<210> 18
<211> 20
<212> DNA
<213> Artificial Sequence

<220>
<223> primer HYG-R

<400> 18
caatgaccgc tgttatgcgg 20

<210> 19
<211> 4666
<212> DNA
<213> Artificial Sequence

<220>
<223> CPR-b and HYG

<400> 19
gggatctcct ccgcagttta tgttcatgtc tttcccactt tggttgtgat tggggtagcg 60

tagtgagttg gtgattttct tttttcgcag gtgtctccga tatcgaagtt tgatgaatat 120

aggagccaga tcagcatggt atattgcctt tgtagataga gatgttgaac aacaactagc 180

tgaattacac accaccgcta aacgatgcgc acagggtgtc accgccaact gacgttgggt 240

ggagttgttg ttggcagggc catattgcta aacgaagaga agtagcacaa aacccaaggt 300

taagaacaat taaaaaaatt catacgacaa ttccacagcc atttacataa tcaacagcga 360

caaatgagac agaaaaaact ttcaacattt caaagttccc tttttcctat tacttctttt 420

tttctttcct tcctttcatt tcctttcctt ctgcttttat tactttacca gtcttttgct 480

tgtttttgca attcctcatc ctcctcctca ccatggcttt agacaagtta gatttgtatg 540

tcatcataac attggtggtc gctgtggccg cctattttgc taagaaccag ttccttgatc 600

agccccagga caccgggttc ctcaacacgg acagcggaag caactccaga gacgtcttgc 660

tgacattgaa gaagaataat aaaaacacgt tgttgttgtt tgggtcccag accggtacgg 720

cagaagatta cgccaacaaa ttgtcaagag aattgcactc cagatttggc ttgaaaacca 780

tggttgcaga tttcgctgat tacgattggg ataacttcgg agatatcacc gaagatatct 840

tggtgttttt catcgttgcc acctacggtg agggtgaacc taccgacaat gccgacgagt 900

tccacacctg gttgactgaa gaagctgaca ctttgagtac tttgagatat accgtgttcg 960

ggttgggtaa ctccacctac gagttcttca atgctattgg tagaaagttt gacagattgt 1020

tgagtgagaa aggtggtgac agatttgctg aatatgctga aggtgacgac ggcactggca 1080

ccttggacga agatttcatg gcctggaagg ataatgtctt tgacgccttg aagaatgact 1140

tgaactttga agaaaaggaa ttgaagtacg aaccaaacgt gaaattgact gagagagatg 1200

acttgtctgc tgccgactcc caagtttcct tgggtgagcc aaacaagaag tacatcaact 1260

ccgagggcat cgacttgacc aagggtccat tcgaccacac ccacccatac ttggccagga 1320

tcaccgagac cagagagttg ttcagctcca aggaaagaca ctgtattcac gttgaatttg 1380

acatttctga atcgaacttg aaatacacca ccggtgacca tctagccatc tggccatcca 1440

actccgacga aaacatcaag caatttgcca agtgtttcgg attggaagat aaactcgaca 1500

ctgttattga attgaaggca ttggactcca cttacaccat tccattccca actccaatta 1560

cttacggtgc tgtcattaga caccatttag aaatctccgg tccagtctcg agacaattct 1620

ttttgtcgat tgctgggttt gctcctgatg aagaaacaaa gaagactttc accagacttg 1680

gtggtgacaa acaagaattc gccaccaagg ttacccgcag aaagttcaac attgccgatg 1740

ccttgttata ttcctccaac aacactccat ggtccgatgt tccttttgag ttccttattg 1800

aaaacatcca acacttgact ccacgttact actccatttc ttcttcgtcg ttgagtgaaa 1860

aacaactcat caatgttact gcagtcgttg aggccgaaga agaagccgat ggcagaccag 1920

tcactggtgt tgttaccaac ttgttgaaga acattgaaat tgcgcaaaac aagactggcg 1980

aaaagccact tgttcactac gatttgagcg gcccaagagg caagttcaac aagttcaagt 2040

tgccagtgca cgtgagaaga tccaacttta agttgccaaa gaactccacc accccagtta 2100

tcttgattgg tccaggtact ggtgttgccc cattgagagg tttcgttaga gaaagagttc 2160

aacaagtcaa gaatggtgtc aatgttggca agactttgtt gttttatggt tgcagaaact 2220

ccaacgagga ctttttgtac aagcaagaat gggccgagta cgcttctgtt ttgggtgaaa 2280

actttgagat gttcaatgcc ttctctagac aagacccatc caagaaggtt tacgtccagg 2340

ataagatttt agaaaacagc caacttgtgc acgaattgtt gaccgaaggt gccattatct 2400

acgtctgtgg tgacgccagt agaatggcca gagacgtcca gaccacgatc tccaagattg 2460

ttgccaaaag cagagaaatc agtgaagaca aggccgctga attggtcaag tcctggaaag 2520

tccaaaatag ataccaagaa gatgtttggt agactcaaac gaatctctct ttctcccaac 2580

gcatttatga atattctcat tgaagtttta catatgttct atatttcatt ttttttttat 2640

tatattacga aacataggtc aactatatat acttgattaa atgttataga aacaataatt 2700

attatctact cgtctacttc tttggcattg gcattggcat tggcattggc attgccgttg 2760

ccgttggtaa tgccgggata gcatgcgaac ccgaaaatgg agcaatcttc cccggggcct 2820

ccaaatacca actcacccga gagagataaa gagacaccac ccaccacgag acggagtata 2880

tccaccaagg taagtaactc agagttaatg atacaggtgt acacagctcc ttccctagcc 2940

attgagtggg tatcacatga cactggtagg ttacaaccac gtttagtagt tattttgtgc 3000

aattccatgg ggatcaggaa gtttggtttg gtgggtgcgt ctactgattc ccctttgtct 3060

ctgaaaatct tttccctagt ggaacacttt ggctgaatga tataaattca ccttgattcc 3120

caccctccct tctttctctc tctctctgtt acacccaatt gaattttctt ttttttttta 3180

ctttccctcc ttctttatca tcaaagataa gtaagtttat caattgccta ttcagaatga 3240

aaaagcctga actcaccgcg acgtctgtcg agaagtttct catcgaaaag ttcgacagcg 3300

tctccgacct catgcagctc tcggagggcg aagaatctcg tgctttcagc ttcgatgtag 3360

gagggcgtgg atatgtcctc cgggtaaata gctgcgccga tggtttctac aaagatcgtt 3420

atgtttatcg gcactttgca tcggccgcgc tcccgattcc ggaagtgctt gacattgggg 3480

aattcagcga gagcctcacc tattgcatct cccgccgtgc acagggtgtc acgttgcaag 3540

acctccctga aaccgaactc cccgctgttc tccagccggt cgcggaggcc atggatgcga 3600

tcgctgcggc cgatcttagc cagacgagcg ggttcggccc attcggaccg caaggaatcg 3660

gtcaatacac tacatggcgt gatttcatat gcgcgattgc tgatccccat gtgtatcact 3720

ggcaaactgt gatggacgac accgtcagtg cgtccgtcgc gcaggctctc gatgagctca 3780

tgctttgggc cgaggactgc cccgaagtcc ggcacctcgt gcacgcggat ttcggctcca 3840

acaatgtcct cacggacaat ggccgcataa cagcggtcat tgactggagc gaggcgatgt 3900

tcggggattc ccaatacgag gtcgccaaca tcttcttctg gaggccgtgg ttggcttgta 3960

tggagcagca gacgcgctac ttcgagcgga ggcatccgga gcttgcagga tcgccgcggc 4020

tccgggcgta tatgctccgc attggtcttg accaactcta tcagagcttg gttgacggca 4080

atttcgatga tgcagcttgg gcgcagggtc gatgcgacgc aatcgtccga tccggagccg 4140

ggactgtcgg gcgtacacaa atcgcccgca gaagcgcggc cgtctggacc gatggctgtg 4200

tagaagtact cgccgatagt ggaaaccgac gccccagcac tcgtccgagg gcaaaggaat 4260

agtgtgctac ccacgcttac tccaccagag ctattaacat cagaaatatt tattctaata 4320

aataggatgc aaaaaaaaaa ccccccttaa taaaaaaaaa agaaacgatt ttttatctaa 4380

tgaagtctat gtatctaaca aatgtatgta tcaatgttta ttccgttaaa caaaaatcag 4440

tctgtaaaaa aggttctaaa taaatattct gtctagtgta cacattctcc caaaatagtg 4500

aaatccagct gctagcttta gtacagtatc tccaatccgg atttgagcta ttgtaaatca 4560

gctgcaagtc attctccacc ttcaaccagt acttatactt catctttgac ttcaagtcca 4620

agtcataaat attacaagtt agcaagaact tctggccatc cacaat 4666

<210> 20
<211> 20
<212> DNA
<213> Artificial Sequence

<220>
<223> primer CPR-3-F

<400> 20
gggatctcct ccgcagttta 20

<210> 21
<211> 20
<212> DNA
<213> Artificial Sequence

<220>
<223> primer CPR-3-R

<400> 21
attgtggatg gccagaagtt 20

<210> 22
<211> 3245
<212> DNA
<213> Candida tropicalis

<400> 22
aattagttat ggggggggga tcaactgatt agcggaagat tggtgttgcc tgtggggttc 60

ttttattttt catatgattt ctttgcgcga gtaacatgtg ccaatctagt ttatgattag 120

cgtacctcca caattggcat cttggacggg cgtgttttgt cttaccccaa gccttattta 180

gttccacagt ctcgacggtg tctcgccgat gtcttctccc acccctcgca ggaatcattc 240

gaagttgttg ggggatctcc tccgcagttt atgttcatgt ctttcccact ttggttgtga 300

ttggggtagc gtagtgagtt ggtgattttc ttttttcgca ggtgtctccg atatcgaagt 360

ttgatgaata taggagccag atcagcatgg tatattgcct ttgtagatag agatgttgaa 420

caacaactag ctgaattaca cgccaccgct aaacgatgcg cacagggtgt caccgccaac 480

tgacgttggg tggagttgtt gttggcaggg ccatattgct aaacgaagag aagtagcaca 540
aaacccaagg ttaagaacaa ttaaaaaaat tcatacgaca attccacagc catttacata 600
atcaacagcg acaaatgaga cagaaaaaac tttcaacatt tcaaagttcc ctttttccta 660
ttacttcttt ttttctttcc ttcctttcat ttcctttcct tctgctttta ttactttacc 720
agtcttttgc ttgtttttgc aattcctcat cctcctcctc accatggctt tagacaagtt 780
agatttgtat gtcatcataa cattggtggt cgctgtggcc gcctattttg ctaagaacca 840
gttccttgat cagccccagg acaccgggtt cctcaacacg gacagcggaa gcaactccag 900
agacgtcttg ctgacattga agaagaataa taaaaacacg ttgttgttgt ttgggtccca 960
gaccggtacg gcagaagatt acgccaacaa attgtcaaga gaattgcact ccagatttgg 1020
cttgaaaacc atggttgcag atttcgctga ttacgattgg gataacttcg gagatatcac 1080
cgaagatatc ttggtgtttt tcatcgttgc cacctacggt gagggtgaac ctaccgacaa 1140
tgccgacgag ttccacacct ggttgactga agaagctgac actttgagta ctttgagata 1200
taccgtgttc gggttgggta actccaccta cgagttcttc aatgctattg gtagaaagtt 1260
tgacagattg ttgagtgaga aaggtggtga cagatttgct gaatatgctg aaggtgacga 1320
cggcactggc accttggacg aagatttcat ggcctggaag gataatgtct ttgacgcctt 1380
gaagaatgac ttgaactttg aagaaaagga attgaagtac gaaccaaacg tgaaattgac 1440
tgagagagat gacttgtctg ctgccgactc ccaagtttcc ttgggtgagc caaacaagaa 1500
gtacatcaac tccgagggca tcgacttgac caagggtcca ttcgaccaca cccacccata 1560
cttggccagg atcaccgaga ccagagagtt gttcagctcc aaggaaagac actgtattca 1620
cgttgaattt gacatttctg aatcgaactt gaaatacacc accggtgacc atctagccat 1680
ctggccatcc aactccgacg aaaacatcaa gcaatttgcc aagtgtttcg gattggaaga 1740
taaactcgac actgttattg aattgaaggc attggactcc acttacacca ttccattccc 1800
aactccaatt acttacggtg ctgtcattag acaccattta gaaatctccg gtccagtctc 1860
gagacaattc tttttgtcga ttgctgggtt tgctcctgat gaagaaacaa agaagacttt 1920
caccagactt ggtggtgaca aacaagaatt cgccaccaag gttacccgca gaaagttcaa 1980
cattgccgat gccttgttat attcctccaa caacactcca tggtccgatg ttccttttga 2040
gttccttatt gaaaacatcc aacacttgac tccacgttac tactccattt cttcttcgtc 2100
gttgagtgaa aaacaactca tcaatgttac tgcagtcgtt gaggccgaag aagaagccga 2160
tggcagacca gtcactggtg ttgttaccaa cttgttgaag aacattgaaa ttgcgcaaaa 2220
caagactggc gaaaagccac ttgttcacta cgatttgagc ggcccaagag gcaagttcaa 2280
caagttcaag ttgccagtgc acgtgagaag atccaacttt aagttgccaa agaactccac 2340
caccccagtt atcttgattg gtccaggtac tggtgttgcc ccattgagag gtttcgttag 2400

agaaagagtt caacaagtca agaatggtgt caatgttggc aagactttgt tgttttatgg 2460

ttgcagaaac tccaacgagg actttttgta caagcaagaa tgggccgagt acgcttctgt 2520

tttgggtgaa aactttgaga tgttcaatgc cttctctaga caagacccat ccaagaaggt 2580

ttacgtccag gataagattt tagaaaacag ccaacttgtg cacgaattgt tgaccgaagg 2640

tgccattatc tacgtctgtg gtgacgccag tagaatggcc agagacgtcc agaccacgat 2700

ctccaagatt gttgccaaaa gcagagaaat cagtgaagac aaggccgctg aattggtcaa 2760

gtcctggaaa gtccaaaata gataccaaga agatgtttgg tagactcaaa cgaatctctc 2820

tctctcccaa cgcatttatg aatattctca ttgaagtttt acatatgttc tatatttcat 2880

ttttttttat tatattacga aacataggtc aactatatat acttgattaa atgttataga 2940

aacaataatt attatctact cgtctacttc tttggcattg gcattggcat tggcattggc 3000

attgccgttg ccgttggtaa tgccgggata tttagtacag tatctccaat ccggatttga 3060

gctattgtaa atcagctgca agtcattctc caccttcaac cagtacttat acttcatctt 3120

tgacttcaag tccaagtcat aaatattaca agttagcaag aacttctggc catccacaat 3180

atagacgtta ttcacgttat tatgcgacgt atggatatgg ttatccttat tgaacttctc 3240

aaact 3245

<210> 23
<211> 3246
<212> DNA
<213> Artificial Sequence

<220>
<223> mutated Cpr-b

<400> 23
aattagttat ggggggggga tcaactgatt agcggaagat tggtgttgcc tgtggggttc 60

ttttattttt catatgattt ctttgcgcga gtaacatgtg ccaatctagt ttatgattag 120

cgtacctcca caattggcat cttggacggg cgtgttttgt cttaccccaa gccttattta 180

gttccacagt ctcgacggtg tctcgccgat gtcttctccc acccctcgca ggaatcattc 240

gaagttgttg ggggatctcc tccgcagttt atgttcatgt ctttcccact ttggttgtga 300

ttggggtagc gtagtgagtt ggtgattttc ttttttcgca ggtgtctccg atatcgaagt 360

ttgatgaata taggagccag atcagcatgg tatattgcct ttgtagatag agatgttgaa 420

caacaactag ctgaattaca caccaccgct aaacgatgcg cacagggtgt caccgccaac 480

tgacgttggg tggagttgtt gttggcaggg ccatattgct aaacgaagag aagtagcaca 540

aaacccaagg ttaagaacaa ttaaaaaaat tcatacgaca attccacagc catttacata 600

atcaacagcg acaaatgaga cagaaaaaac tttcaacatt tcaaagttcc ctttttccta 660

ttacttcttt ttttctttcc ttcctttcat ttcctttcct tctgctttta ttactttacc 720

agtcttttgc ttgtttttgc aattcctcat cctcctcctc accatggctt tagacaagtt 780

agatttgtat gtcatcataa cattggtggt cgctgtggcc gcctattttg ctaagaacca 840

gttccttgat cagccccagg acaccgggtt cctcaacacg gacagcggaa gcaactccag 900

agacgtcttg ctgacattga agaagaataa taaaaacacg ttgttgttgt ttgggtccca 960

gaccggtacg gcagaagatt acgccaacaa attgtcaaga gaattgcact ccagatttgg 1020

cttgaaaacc atggttgcag atttcgctga ttacgattgg gataacttcg gagatatcac 1080

cgaagatatc ttggtgtttt tcatcgttgc cacctacggt gagggtgaac ctaccgacaa 1140

tgccgacgag ttccacacct ggttgactga agaagctgac actttgagta ctttgagata 1200

taccgtgttc gggttgggta actccaccta cgagttcttc aatgctattg gtagaaagtt 1260

tgacagattg ttgagtgaga aaggtggtga cagatttgct gaatatgctg aaggtgacga 1320

cggcactggc accttggacg aagatttcat ggcctggaag gataatgtct ttgacgcctt 1380

gaagaatgac ttgaactttg aagaaaagga attgaagtac gaaccaaacg tgaaattgac 1440

tgagagagat gacttgtctg ctgccgactc ccaagtttcc ttgggtgagc caaacaagaa 1500

gtacatcaac tccgagggca tcgacttgac caagggtcca ttcgaccaca cccacccata 1560

cttggccagg atcaccgaga ccagagagtt gttcagctcc aaggaaagac actgtattca 1620

cgttgaattt gacatttctg aatcgaactt gaaatacacc accggtgacc atctagccat 1680

ctggccatcc aactccgacg aaaacatcaa gcaatttgcc aagtgtttcg gattggaaga 1740

taaactcgac actgttattg aattgaaggc attggactcc acttacacca ttccattccc 1800

aactccaatt acttacggtg ctgtcattag acaccattta gaaatctccg gtccagtctc 1860

gagacaattc tttttgtcga ttgctgggtt tgctcctgat gaagaaacaa agaagacttt 1920

caccagactt ggtggtgaca aacaagaatt cgccaccaag gttacccgca gaaagttcaa 1980

cattgccgat gccttgttat attcctccaa caacactcca tggtccgatg ttccttttga 2040

gttccttatt gaaaacatcc aacacttgac tccacgttac tactccattt cttcttcgtc 2100

gttgagtgaa aaacaactca tcaatgttac tgcagtcgtt gaggccgaag aagaagccga 2160

tggcagacca gtcactggtg ttgttaccaa cttgttgaag aacattgaaa ttgcgcaaaa 2220

caagactggc gaaaagccac ttgttcacta cgatttgagc ggcccaagag gcaagttcaa 2280

caagttcaag ttgccagtgc acgtgagaag atccaacttt aagttgccaa agaactccac 2340

caccccagtt atcttgattg gtccaggtac tggtgttgcc ccattgagag gtttcgttag 2400

agaaagagtt caacaagtca agaatggtgt caatgttggc aagactttgt tgttttatgg 2460

ttgcagaaac tccaacgagg actttttgta caagcaagaa tgggccgagt acgcttctgt 2520

tttgggtgaa aactttgaga tgttcaatgc cttctctaga caagacccat ccaagaaggt 2580

ttacgtccag gataagattt tagaaaacag ccaacttgtg cacgaattgt tgaccgaagg 2640

tgccattatc tacgtctgtg gtgacgccag tagaatggcc agagacgtcc agaccacgat 2700

ctccaagatt gttgccaaaa gcagagaaat cagtgaagac aaggccgctg aattggtcaa 2760

gtcctggaaa gtccaaaata gataccaaga agatgtttgg tagactcaaa cgaatctctc 2820

tttctcccaa cgcatttatg aatattctca ttgaagtttt acatatgttc tatatttcat 2880

ttttttttta ttatattacg aaacataggt caactatata tacttgatta aatgttatag 2940

aaacaataat tattatctac tcgtctactt ctttggcatt ggcattggca ttggcattgg 3000

cattgccgtt gccgttggta atgccgggat atttagtaca gtatctccaa tccggatttg 3060

agctattgta aatcagctgc aagtcattct ccaccttcaa ccagtactta tacttcatct 3120

ttgacttcaa gtccaagtca taaatattac aagttagcaa gaacttctgg ccatccacaa 3180

tatagacgtt attcacgtta ttatgcgacg tatggatatg gttatcctta ttgaacttct 3240

caaact 3246

## Description

### Field of the Invention

The invention relates to a long-chain dibasic acid with low content of fatty acid impurity and a method of producing the same; as well as a method for preparing a long-chain dibasic acid producing strain by using directed evolution and homologous recombination, and a method for producing a long-chain dibasic acid with low content of fatty acid impurity by using said strain.

### Background

Long-chain dibasic acid (LCDA; also referred to as long chain dicarboxylic acid or long chain diacid) is a dibasic acid comprising the formula HOOC(CH₂)ₙCOOH, where n≥7. As important monomer raw materials, long-chain dibasic acids are widely used in the synthesis of nylon, resins, hot-melt adhesives, powder coatings, preservatives, perfumes, lubricants, plasticizers, and the like.

For a long time, long-chain dibasic acid is synthesized via classical chemical synthesis pathway from the petroleum, such as butadiene multiple-step oxidation process. The chemical synthesis methods face many challenges. Dibasic acid obtained by the chemical synthesis method is a mixture of long-chain dibasic acid and short-chain dibasic acid, and thus the subsequent complicated extraction and purification steps are necessary, which becomes a huge obstacle for production techniques and production cost. Microbiological fermentation technology for producing a long-chain dibasic acid is advantageous over classical chemical synthesis method because it produces less pollution, is environment friendly, and is capable of synthesizing a long-chain dibasic acid such as the one having more than 12 carbon atoms which is difficult to be produced by chemical synthesis methods and has high purity and the like.

But using the microbiological fermentation method for producing a long-chain dibasic acid, there are residual impurities in the products sometimes and the reduction in the product purity affects the quality of the product significantly, which greatly affects its later application. In particular, the impurity which is characteristically similar to the long-chain dibasic acid not only generates technical challenges to the later extraction and purification, but also produces great negative effects on the production cost control. Therefore, to genetically modify a strain for producing a long-chain dibasic acid so as to reduce the content of certain impurity during the fermentation is important and valuable in industry to the dibasic acid production via biological synthesis.

Previously, the improvement of a dibasic acid producing strain was mostly achieved through conventional random mutagenesis or genetic engineering methods. Due to the characteristic of random mutagenesis, there is a high requirement for screening throughput, and a new round of mutagenesis screening is required for each changed trait, which has become an important limiting factor technically. The genetic engineering means can be used to perform targeted genetic modification of a strain, so as to obtain a good strain with higher yield. The microbiological fermentation method of a long-chain dibasic acid is mainly based on ω-oxidation of alkane, which can then be degraded by β-oxidation pathway. Previous studies have shown that the yield of a long-chain dibasic acid can be increased by means of enhancing the ω-oxidation pathway and inhibiting the β-oxidation pathway. Pictaggio et al. of Coginis company (Mol. Cell. Biol., 11(9), 4333-4339, 1991) reported that knocking out two alleles of each of POX4 and POX5 could effectively block the β-oxidation pathway to achieve 100% conversion rate of the substrate. Further overexpression of the genes of two key enzymes, P450 and oxidoreductase CPR-b, of the rate-limiting step in the ω-oxidation pathway could effectively increase production. Xiaoqin Lai *et al.* (Chinese patent CN103992959B) reported that the introduction of one copy of the CYP52A14 gene into a dibasic acid-producing strain could also effectively increase the conversion rate and production efficiency of the dibasic acid. In addition, Zhu'an Cao et al. (Biotechnol. J., 1, 68-74, 2006) from Tsinghua University found that knocking out a copy of the key gene CAT in the transportation of acetyl coenzyme A from peroxisome to mitochondria could partially block acetyl coenzyme A entering the citric acid cycle, and also effectively reduce the degradation of dibasic acids.

Error-prone PCR is the technique proposed by Leung et al. (Technical, 1, 11-15, 1989) to construct a gene library for directed studies. By changing PCR conditions, such as adjusting the concentration of four deoxyribonucleic acids in the reaction system, changing the concentration of Mg²⁺, and using a low-fidelity DNA polymerase and the like, the bases are mismatched so as to introduce a mutation. Too high or too low mutation rate will affect the effect of constructing mutant libraries. The ideal base mutation ratio is 1-3 per DNA fragment. Therefore, the beneficial mutations that contribute to further improvement of the strain productivity can be screened out through gene-directed genetic modification by using error-prone PCR of generating random mutations in combination with homologous recombination.

Nonetheless, it has not been reported that a dibasic acid producing strain is modified by means of genetic engineering to reduce the content of fatty acids. There is still a need in the art for a long-chain dibasic acid product with low content of impurity, as well as a strain for producing such a product by fermentation and a preparation method thereof.

### Summary of the Invention

The invention relates to an isolated mutated CPR-b gene, homologous gene or variant thereof, relative to the GenBank Accession Number AY823228 (e.g. set forth in SEQ ID NO: 22), taking the first base upstream of the start codon ATG (e.g. the base "C" at position 763 of SEQ ID NO: 22) as -1, comprising one base mutation -322G>A in its promoter region, and taking the first base downstream of the stop codon TAG (e.g. the base "A" at position 2804 of SEQ ID NO: 22) as 1, comprising mutations 3'UTR.19C>T and 3'UTR.76_77insT in its terminator region; and the variant has at least 70% sequence identity to the mutated CPR-b gene or homologous gene thereof.

In some embodiments, the sequence of the mutated CPR-b gene is set forth in SEQ ID NO: 13 or 23, or has at least 70%, e.g. at least or at least about 75%, 80%, 85%, 90%, 91%, 92%, 93%, 94%, 95%, 96%, 97%, 98%, 99%, 99.18%, 99.21%, 99.25%, 99.28%, 99.32%, 99.36%, 99.39%, 99.43%, 99.46%, 99.50%, 99.53%, 99.57%, 99.61%, 99.64%, 99.68%, 99.72%, 99.75%, 99.79%, 99.82%, 99.86%, 99.89%, 99.93% or 99.96%, sequence identity thereto.

The invention further relates to a microorganism containing the mutated CPR-b gene, homologous gene or variant thereof according to the invention, which produces a long-chain dibasic acid with reduced content of a fatty acid impurity, compared to a microorganism containing a non-mutated CPR-b gene or homologous gene thereof.

The invention further relates to a method of producing a long-chain dibasic acid by fermentation with a microorganism containing the mutated CPR-b gene, homologous gene or variant thereof according to the invention, comprising a step of culturing the microorganism, and optionally a step of isolating, extracting and/or purifying the long-chain dibasic acid from the culture.

In some embodiments, after the completion of the process of producing a long-chain dibasic acid by fermentation with a microorganism according to the invention, the fermentation broth contains fatty acid impurity, and the mass ratio of the fatty acid impurity is below 1.50%, wherein the mass ratio is the mass percentage of the fatty acid impurity to the long-chain dibasic acid in the fermentation broth.

In some embodiments, after the completion of the process of producing a long-chain dibasic acid by fermentation with a microorganism according to the invention, the fermentation broth contains fatty acid impurity, and compared with the content of the fatty acid impurity in the long-chain dibasic acid produced by fermentation method with a conventional microorganism, such as by fermentation with the non-mutant microorganism according to the invention, the fatty acid impurity in the fermentation broth is decreased by at least 5%.

The invention further relates to a long-chain dibasic acid with low content of fatty acid impurity, wherein the content of the fatty acid impurity contained in the long-chain dibasic acid is more than 0 and less than 4,000 ppm, preferably less than 1000 ppm, more preferably less than 200 ppm, and the fatty acid impurity comprises a saturated linear organic acid with one terminal carboxyl group. Preferably, the long-chain dibasic acid is produced by fermentation of a long-chain dibasic acid producing microorganism strain.

In some embodiments, the long-chain dibasic acid producing microorganism strain contains the mutated CPR-b gene, homologous gene or variant thereof according to the invention. In some embodiments, the long-chain dibasic acid producing microorganism strain is the microorganism according to the invention which contains the mutated CPR-b gene, homologous gene or variant thereof according to the invention.

In some embodiments, the microorganism of the invention is selected from the group consisting of *Corynebacterium, Geotrichum candidum, Candida, Pichia, Rhodotroula, Saccharomyces* and *Yarrowia,* more preferably the microorganism is yeast, and more preferably the microorganism is *Candida tropicalis* or *Candida sake.* In a particular embodiment, the microorganism is CCTCC M2011192 or CCTCC M203052.

In some embodiments, the long-chain dibasic acid is selected from C9 to C22 long-chain dibasic acids, preferably selected from C9 to C18 long-chain dibasic acids, more preferably one or more selected from the group consisting of C10 dibasic acid, C11 dibasic acid, C12 dibasic acid, C13 dibasic acid, C14 dibasic acid, C15 dibasic acid and C16 dibasic acid. More preferably, the long-chain dibasic acid is at least one of C10 to C16 dibasic acids, or at least one of *n*-C10 to C16 dibasic acids, e.g. at least one selected from the group consisting of sebacic acid, undecanedioic acid, dodecanedioic acid, tridecanedioic acid, tetradecanedioic acid, pentadecanedioic acid and hexadecanedioic acid.

In some embodiments, the fatty acid impurity has the formula CH₃-(CH₂)ₙ-COOH, where n≥7, preferably the fatty acid impurity comprises a long-chain fatty acid with the number of carbon atoms in the carbon chain being greater than 9 and with one terminal carboxyl group; preferably the fatty acid impurity comprises any one or more of C9 fatty acid, C10 fatty acid or capric acid, C11 fatty acid, C12 fatty acid or lauric acid, C13 fatty acid, C14 fatty acid or myristic acid, C15 fatty acid, C16 fatty acid or palmitic acid, C17 fatty acid, C18 fatty acid or stearic acid, or C19 fatty acid.

In some embodiments, where the long-chain dibasic acid is C12 dibasic acid such as dodecanedioic acid, the fatty acid impurity is predominantly lauric acid, and the content of the lauric acid impurity is less than 3000 ppm, preferably less than 400 ppm, 300 ppm, 200ppm, or less.

In some embodiments, where the long-chain dibasic acid is C10 dibasic acid such as sebacic acid, the fatty acid impurity is predominantly capric acid, and the content of the capric acid impurity is less than 2000 ppm, preferably less than 500 ppm, 400 ppm, 300 ppm, 200 ppm, or less.

In some embodiments, where the long-chain dibasic acid is C16 dibasic acid such as hexadecanedioic acid, the fatty acid impurity is predominantly palmitic acid, and the content of the palmitic acid impurity is less than 4000 ppm, preferably less than 500 ppm, 400 ppm, 300ppm, or less.

The invention further relates to a method of modifying a long-chain dibasic acid producing microorganism strain, comprising a step of direct-evolution of a key gene in the pathway of the long-chain dibasic acid synthesis, wherein, compared to the microorganism strain before modified, the modified long chain dibasic acid producing microorganism strain is capable of producing the long chain dibasic acid with substantially decreased content of fatty acid impurity, e.g. under the same conditions. In some embodiments, the key gene in the pathway of the long-chain dibasic acid synthesis is CPR-b gene.

In some embodiments, the microorganism of the invention is selected from the group consisting of *Corynebacterium, Geotrichum candidum, Candida, Pichia, Rhodotroula, Saccharomyces* and *Yarrowia,* more preferably the microorganism is yeast, and more preferably the microorganism is *Candida tropicalis* or *Candida sake.* In a particular embodiment, the microorganism is CCTCC M2011192 or CCTCC M203052.

In some embodiments, the long-chain dibasic acid according to the invention is selected from C9 to C22 long-chain dibasic acids, preferably selected from C9 to C18 long-chain dibasic acids, more preferably one or more selected from the group consisting of C10 dibasic acid, C11 dibasic acid, C12 dibasic acid, C13 dibasic acid, C14 dibasic acid, C15 dibasic acid and C16 dibasic acid. More preferably, the long-chain dibasic acid is at least one of C10 to C16 dibasic acids, or at least one of *n*-C10 to C16 dibasic acids, e.g. at least one selected from the group consisting of sebacic acid, undecanedioic acid, dodecanedioic acid, tridecanedioic acid, tetradecanedioic acid, pentadecanedioic acid and hexadecanedioic acid.

In some embodiments, the fatty acid impurity according to the invention comprises those with the number of carbon atoms in the carbon chain being greater than 9, preferably C10 acid (capric acid), C12 acid (lauric acid), C14 acid (myristic acid), C16 acid (palmitic acid), and/or C18 acid (stearic acid). Preferably, the content of the fatty acid impurity is decreased to below 300 ppm, e.g. below 290ppm, 270ppm, 250ppm, 200ppm, 150ppm, 140ppm, 130ppm, 120ppm, 110ppm, 100ppm or lower.

In some embodiments, the method of modifying a long-chain dibasic acid producing microorganism strain comprises steps of:
1) preparing a target gene fragment having a mutation by error-prone PCR;
2) preparing fragments upstream and downstream of the target gene necessary for homologous recombination as templates for homologous recombination with a resistance marker gene, preferably the resistance marker gene is hygromycin B;
3) preparing a complete recombination fragment by PCR overlap extension;
4) introducing the recombination fragment into a strain by homologous recombination;
5) screening positive strains by means of the resistance marker;
6) screening strains wherein the content of fatty acid impurity in the fermentation broth after completion of fermentation is significantly decreased;
7) optionally, removing the resistance marker in the screened strains by further homologous recombination.

The invention further relates to relates to a fermentation broth in a process of producing a long-chain dibasic acid by fermentation with a microorganism, wherein the fermentation broth contains a fatty acid impurity, and the content of the fatty acid impurity is below 1.5%, such as below 1.4%, 1.3%, 1.2 %, 1.1%, 1.0%, 0.9%, 0.8%, 0.7%, 0.6%, 0.5%, 0.4%, 0.3% or lower, wherein the percentage is the mass percentage of the fatty acid impurity to the long-chain dibasic acid in the fermentation broth.

Preferably, the long-chain dibasic acid is selected from C9 to C22 long-chain dibasic acids, and the fatty acid impurity comprises a saturated linear organic acid with one terminal carboxyl group.

In some embodiments, the microorganism contains the mutated CPR-b gene, homologous gene or variant thereof according to the invention. In some embodiments, the microorganism is the microorganism according to the invention which contains the mutated CPR-b gene, homologous gene or variant thereof according to the invention. In some embodiments, the fermentation broth is obtained by the present inventive method of producing a long-chain dibasic acid by fermentation with a microorganism containing the mutated CPR-b gene, homologous gene or variant thereof according to the invention. In some embodiments, the fermentation broth is obtained in a process of producing a long-chain dibasic acid by using a microorganism obtained by the method of modifying a long-chain dibasic acid producing microorganism strain according to the invention.

The invention further relates to relates to a method of producing a long-chain dibasic acid, comprising obtaining a long-chain dibasic acid producing microorganism strain containing a mutated CPR-b gene, homologous gene or a variant thereof by directed evolution of the CPR-b gene in the long-chain dibasic acid synthesis pathway; culturing the strain to produce the long-chain dibasic acid by fermentation; optionally further comprising a step of isolating, extracting and/or purifying the long-chain dibasic acid from the culture product.

The mutated CPR-b gene, homologous gene or variant thereof, relative to the GenBank Accession Number AY823228 (e.g. set forth in SEQ ID NO: 22), taking the first base upstream of the start codon ATG (e.g. the base "C" at position 763 of SEQ ID NO: 22) as -1, comprises one base mutation -322G>A in its promoter region, and taking the first base downstream of the stop codon TAG (e.g. the base "A" at position 2804 of SEQ ID NO: 22) as 1, comprises mutations 3'UTR.19C>T and 3'UTR.76_77insT in its terminator region; and the variant has at least 70% sequence identity to the mutated CPR-b gene or homologous gene thereof.

Preferably, the sequence of the mutated CPR-b gene is set forth in SEQ ID NO. 13 or 23, or has at least 70%, e.g. at least about 75%, 80%, 85%, 90%, 91%, 92%, 93%, 94%, 95%, 96%, 97%, 98%, 99%, 99.18%, 99.21%, 99.25%, 99.28%, 99.32%, 99.36%, 99.39%, 99.43%, 99.46%, 99.50%, 99.53%, 99.57%, 99.61%, 99.64%, 99.68%, 99.72%, 99.75%, 99.79%, 99.82%, 99.86%, 99.89%, 99.93% or 99.96% sequence identity thereto.

In some embodiments, the long-chain dibasic acid is selected from C9-C22 long-chain dibasic acids, preferably selected from C9-C18 long-chain dibasic acids, more preferably one or more selected from the group consisting of C10 dibasic acid, C11 dibasic acid, C12 dibasic acid, C13 dibasic acid, C14 dibasic acid, C15 dibasic acid and C16 dibasic acid. In some embodiments, the long-chain dibasic acid is at least one of C10 to C16 dibasic acids, or at least one of *n*-C10 to C16 dibasic acids, e.g. at least one selected from the group consisting of sebacic acid, undecanedioic acid, dodecanedioic acid, tridecanedioic acid, tetradecanedioic acid, pentadecanedioic acid and hexadecanedioic acid.

In some embodiments, the chemical formula of the fatty acid impurity is CH₃-(CH₂)ₙ-COOH, where n≥7, preferably the fatty acid impurity comprises a long-chain fatty acid having the number of carbon atoms in the carbon chain being greater than 9 and one terminal carboxyl group.

In some embodiments, the microorganism is yeast; more preferably the microorganism is selected from *Candida tropicalis* or *Candida sake.*

In some embodiments, the obtaining a long-chain dibasic acid producing microorganism strain containing a mutated CPR-B gene, a homologous gene or a variant thereof, comprises the following steps:
1) preparing a target (CPR-b) gene fragment having a mutation by error-prone PCR;
2) preparing fragments upstream and downstream of the target (CPR-b) gene necessary for homologous recombination as templates for homologous recombination with a resistance marker gene, preferably the resistance marker gene is hygromycin B;
3) preparing a complete recombination fragment by PCR overlap extension;
4) introducing the recombination fragment into a strain by homologous recombination;
5) screening positive strains by means of the resistance marker;
6) screening strains wherein the content of fatty acid impurity in the fermentation broth after completion of fermentation is significantly decreased;
7) optionally, removing the resistance marker in the screened strains by further homologous recombination.

In the present invention, the *Candida tropicalis* strain CATN145 (Deposit No. CCTCC M2011192) is used as the starting strain, and error-prone PCR method is used to randomly mutate the CPR-b gene. The gene is subjected to directed evolution by homologous recombination method to screen out a long-chain dibasic acid producing strain with significantly decreased content of fatty acid impurity. By screening, the present invention obtains a strain wherein the content of fatty acid impurity in the fermentation product is significantly decreased, and is named as mutant strain 5473. By sequencing analysis, it is found that, compared to the parental strain CCTCC M2011192, taking the first base upstream of the start codon ATG as -1, one base mutation -322G>A is present in the promoter region of the CPR-b gene of the screened *Candida tropicalis* mutant strain of the present invention; and taking the first base downstream of the stop codon TAG as 1, base mutations 3'UTR.19C>T and 3'UTR.76_77insT are present in its terminator region.

According to the present invention, the sequence of the mutated *Candida tropicalis* CPR-b gene comprises or is set forth in SEQ ID: 13.

After further removing the resistance marker from the mutant strain, compared to the parental strain, the mass ratio of fatty acid impurity in the fermentation broth after completion of fermentation is significantly decreased, and the content of the fatty acid impurity in the long-chain dibasic acid product obtained after extracting and purifying the fermentation broth can be decreased to less than 300 ppm.

The present invention screens out a strain which has base mutations in the promoter and terminator regions of the CPR-b gene by directed evolution of said gene, and the content of fatty acid impurity in the fermentation broth is significantly decreased for different fermentation substrates. Compared to the parental strain, the fatty acid content is decreased by almost 40%, and the purity of the fermentation product long-chain dibasic acid is further improved, making the dibasic acid product which is used as important raw materials for nylon filament, engineering plastic, synthetic fragrance, cold-resistant plasticizer, advanced lubricants and polyamide hot melt adhesives, favorable for the production of and the quality improvement of downstream products. More importantly, this greatly reduces the difficulty of the extraction and purification processes at later stages of dibasic acid production, simplifies the process and saves energy.

### Brief Description of the Figures

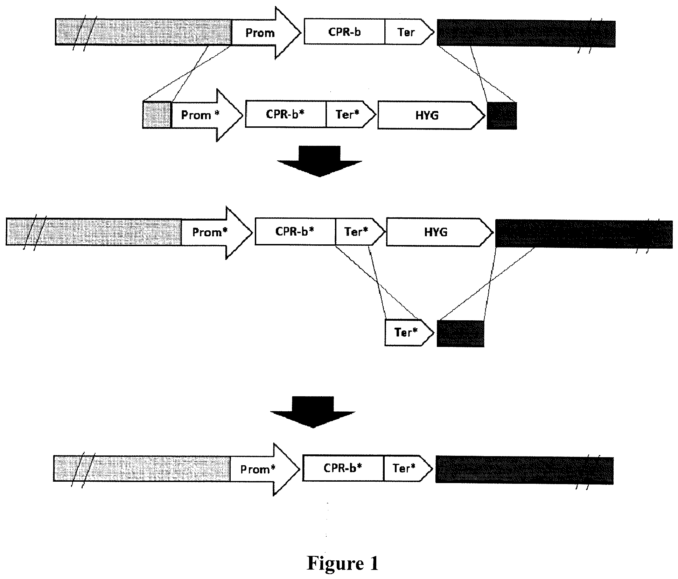

Figure1 is a scheme of the integration of the CPR-b gene with mutations and the removal of the hygromycin resistance marker by homologous recombination. "*" indicates the mutations that may be present in any region of CPR-b (including the promoter, coding region, and terminator).

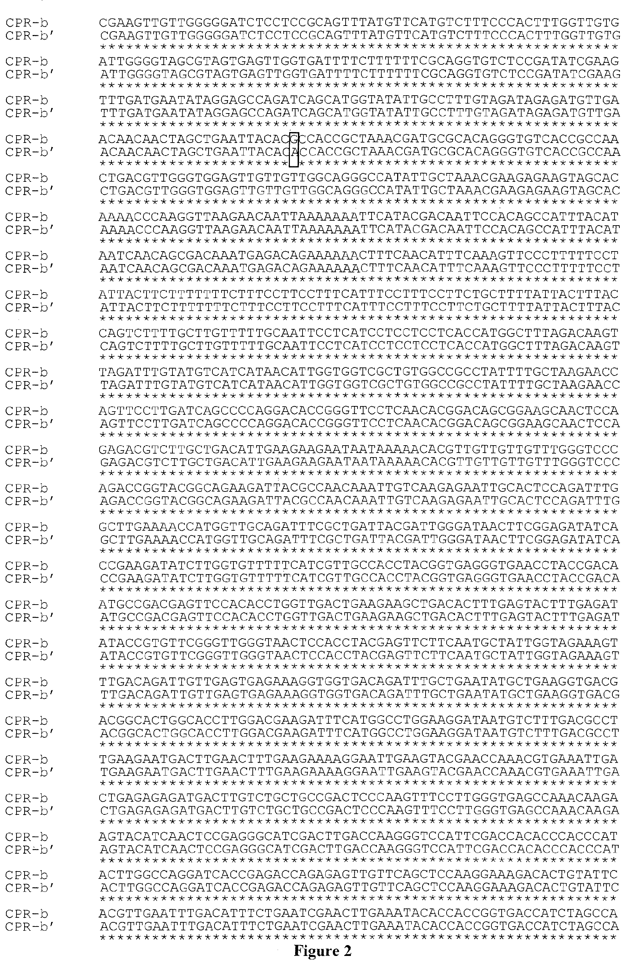

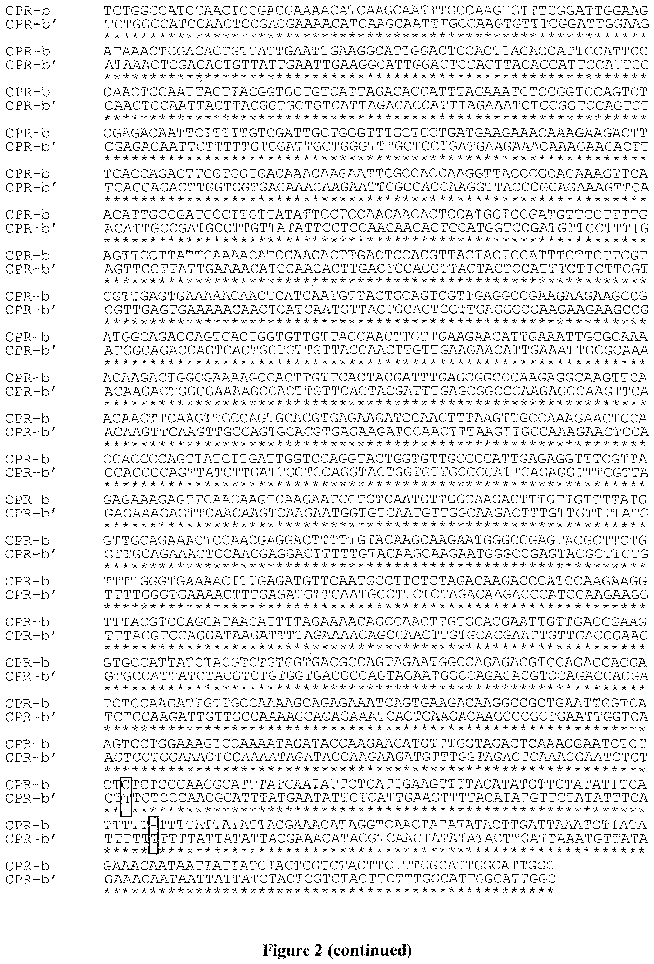

Figure 2 is the alignment of the nucleotide sequences of the CPR-b genes of the mutant strain of the invention (indicated by CPR-b', as set forth in nucleotides 295-3087 of SEQ ID NO: 23) and the original strain (indicated by CPR-b, as set forth in nucleotides 295-3086 of SEQ ID NO: 22), and the mutation sites are boxed with a black box.

### Detailed Description

### Definition

Unless defined otherwise, technical and scientific terms used herein have the same meanings as commonly understood by skilled persons in the art. See e.g. Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, 1989).

Long chain alkane: the fermentation substrate of the invention comprises a long chain alkane, belonging to a saturated aliphatic hydrocarbon, which is a saturated hydrocarbon among hydrocarbons; its whole structure is mostly composed only of carbon, hydrogen, carbon-carbon single bond, and carbon-hydrogen single bond. It includes an alkane of the formula CH₃(CH₂)ₙCH₃, where n≥7. Preferred are C9-C22 normal alkanes, more preferred are C9-C18 normal alkanes, and most preferred are C10, C11, C12, C13, C14, C15 or C16 normal alkanes.

Long-chain dibasic acid (LCDA; also known as long chain dicarboxylic acid or long chain diacid, hereinafter abbreviated as dibasic acid sometimes) includes a dibasic acid of the formula HOOC(CH₂)ₙCOOH, where n≥7. Preferably, the long-chain dibasic acid is selected from C9-C22 long-chain dibasic acids, preferably C9-C18 long-chain dibasic acids; more preferably comprises one or more of C10 dibasic acid, C11 dibasic acid, C12 dibasic acid, C13 dibasic acid, C14 dibasic acid, C15 dibasic acid and C16 dibasic acid. Preferably, the long-chain dibasic acid is at least one of C10 to C16 dibasic acids, and preferably at least one of *n*-C10 to C16 dibasic acids, preferably at least one selected from the group consisting of sebacic acid, undecanedioic acid, dodecanedioic acid, tridecanedioic acid, tetradecanedioic acid, pentadecanedioic acid and hexadecanedioic acid.

Long-chain dibasic acid producing microorganism: the strain that has been reported to produce and accumulate a dibasic acid includes bacterium, yeast, and mold, such as *Corynebacterium, Geotrichum candidum, Candida, Pichia, Rhodotroula, Saccharomyces, Yarrowia,* and the like. Among them, many species of *Candida* are good strains for the production of a dibasic acid by fermentation. The strain for fermentation preferably includes: *Candida tropicalis* and *Candida sake.*

In the process of producing a long-chain dibasic acid by fermentation of a long-chain alkane substrate, alkane is first oxidized to fatty acid and then oxidized to a dibasic acid, but the inventors have found that if the alkane is not completely oxidized, partial of the fatty acid remains in the fermentation broth. Because of its very similar properties to the long-chain dibasic acid, it is difficult to isolate efficiently by conventional means. Fatty acid as impurity will enter the final dibasic acid product along with the subsequent treatment process, greatly affecting the purity and quality of the product.

The fatty acid impurity of the present invention comprises a saturated linear organic acid with one terminal carboxyl group (-COOH). The chemical formula of the fatty acid impurity is CH₃-(CH₂)ₙ-COOH, where n≥7. Preferably, the fatty acid impurity comprises a long-chain fatty acid with the number of carbon atoms in the carbon chain being greater than 9 and with one terminal carboxyl group, such as any one or more of C9 fatty acid, C10 fatty acid or capric acid, C11 fatty acid, C12 fatty acid or lauric acid, C13 fatty acid, C14 fatty acid or myristic acid, C15 fatty acid, C16 fatty acid or palmitic acid, C17 fatty acid, C18 fatty acid or stearic acid, or C19 fatty acid.

As used herein, the expression "substantially or significantly decreased content of fatty acid impurity" refers to that, compared to a reference, the content of the fatty acid impurity is decreased by at least 5%, 6%, 7%, 8%, 9%, 10%, 12%, 14%, 16%, 18%, 20%, 25%, 30%, 35% 40%, 50%, 60%, 70%, 80%, 90%, 95% or more, preferably at least 10%, more preferably at least 20%, more preferably at least 40%, more preferably at least 50%, more preferably at least 70% or more.

When a long-chain dibasic acid is produced by fermentation according to the present invention, the fermentation broth after fermentation contains a fatty acid impurity, and the content of the fatty acid impurity is significantly decreased relative to the content of the fatty acid impurity produced by a conventional microbiological fermentation, such as the fermentation by a non-mutant microorganism described in the present invention, such as by at least 5%, 6%, 7%, 8%, 9%, 10%, 12%, 14%, 16%, 18%, 20%, 25%, 30%, 35% 40%, 50%, 60%, 70%, 80%, 90%, 95% or more, preferably at least 10%, more preferably at least 20%, more preferably at least 40%, more preferably at least 50%, more preferably at least 70% or more.

In some embodiments, the long-chain dibasic acid is produced by a microbiological fermentation, and the fermentation broth contains a fatty acid impurity, and the content of the fatty acid impurity is decreased to below 1.5%, preferably below 1.1%, more preferably below 1.0%, and more preferably below 0.9%, such as below 1.4%, 1.3%, 1.2%, 1.1%, 1.0%, 0.9%, 0.8%, 0.7%, 0.6%, 0.5%, 0.4%, 0.3% or lower, wherein the percentage is a mass percentage of the fatty acid impurity to the long-chain dibasic acid in the fermentation broth.

In some embodiments of the present invention, the long-chain dibasic acid produced by the microbiological fermentation method of the present invention contains a fatty acid impurity, and the content of the fatty acid impurity is below 4,000 ppm, more preferably below 3,000 ppm, below 2000 ppm, below 1000 ppm, below 290 ppm, 270 ppm, 250 ppm, 200 ppm, 150 ppm, 100 ppm or lower.

The unit ppm of the impurity content of the present invention is the mass ratio of the impurity to the long-chain dibasic acid, and 100 ppm = 100^{∗}10⁻⁶= 0.01%. In some embodiments, the impurity of DC16 (C16 dibasic acid), is generally higher than that of DC12 (C12 dibasic acid) and DC10 (C10 dibasic acid) on the whole, such as by at least 10%, at least 20%, at least 30%, at least 40%, at least 50%, 60%, at least 80%, at least 100% or higher, wherein DC refers to long-chain dibasic acid.

In some embodiments of the present invention, when C12 dibasic acid is produced by the microbiological fermentation method, the fatty acid impurity is predominantly lauric acid, and the content of the lauric acid impurity is less than 3000 ppm, preferably less than 500 ppm, 400 ppm, 300 ppm, 200ppm or less.

In some embodiments of the present invention, when C10 dibasic acid is produced by the microbiological fermentation method, the fatty acid impurity is predominantly capric acid, and the content of the capric acid impurity is less than 2000 ppm, preferably less than 500 ppm, 400 ppm, 300 ppm, 200 ppm or less.

In some embodiments of the present invention, when C16 dibasic acid is produced by the microbiological fermentation method, the fatty acid impurity is predominantly palmitic acid, and the content of the palmitic acid impurity is less than 4000 ppm, preferably less than 500 ppm, 400 ppm, 300ppm or less.

The test method for the dibasic acid and the impurity content may employ the techniques well known to those skilled in the art, such as an internal standard method or a normalization method of gas chromatography detection.

CPR-b gene (with Accession Number AY823228 in the GenBank) encodes NADPH-dependent cytochrome reductase that forms a complex with P450 cytochrome oxidase during ω-oxidation and binds to the endoplasmic reticulum membrane, providing electron to P450 as electron donor. It is known to those skilled in the art that the CPR-b gene or homologous gene thereof is also present in other microorganisms producing a long-chain dibasic acid, and the sequences may differ, but are also within the scope of the present invention.

The term "isolated", when applied to a nucleic acid or protein, means that the nucleic acid or protein is essentially free of other cellular components with which it is associated in the natural state. It can be, for example, in a homogeneous state and may be in either a dry or aqueous solution. Purity and homogeneity are typically determined using analytical chemistry techniques such as polyacrylamide gel electrophoresis or high performance liquid chromatography.

As used herein, the expression "relative to the GenBank Accession Number AY823228" refers to a mutation at a corresponding position when aligned with the sequence of AY823228 (SEQ ID NO: 22). The corresponding position refers to the numbering of the residue of the reference sequence (SEQ ID NO: 22) when the given polynucleotide sequence (e.g. a mutated CPR-b gene sequence) is compared to the reference sequence. A base in a nucleic acid "corresponds" to a given base when it occupies the same essential structural position within the nucleic acid as the given base. In general, to identify corresponding positions, the sequences of nucleic acids are aligned so that the highest order match is obtained (see, e.g. Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carillo et al. (1988) SIAM J Applied Math 48: 1073). Alignment of nucleotide sequences also can take into account conservative differences and/or frequent substitutions in nucleotides. Conservative differences are those that preserve the physico-chemical properties of the residues involved. Alignments can be global (alignment of the compared sequences over the entire length of the sequences and including all residues) or local (alignment of a portion of the sequences that includes only the most similar region(s)).

As used herein, the base mutation "XXX NO>N1" means that the base N0 at position XXX is mutated to the base N1. For example, "taking the first base upstream of the start codon ATG as -1, the base mutation -322G>A" means that, taking the first base upstream immediately adjacent to the base "A" of the starting codon ATG as -1, the base "G" at position -322 is mutated to "A", and "taking the first base downstream of the stop codon TAG as 1, the mutations 3'UTR.19C>T and 3'UTR.76_77insT" means that, taking the first base downstream immediately adjacent to the base "G" of the stop codon TAG as 1, the base "C" at position 19 is mutated to "T", and a base "T" is inserted between the bases at positions 76 and 77.

In an embodiment, the sequence of the CPR-b gene according to the invention is set forth in SEQ ID NO: 22, wherein the protein coding sequences are the nucleotides 764 to 2803. Correspondingly, the mutation "-322G>A" correspond to the nucleotide "G" at position 422 of SEQ ID NO: 22 is mutated to "A", the mutation "3'UTR.19C>T" correspond to the nucleotide "C" at position 2822 of SEQ ID NO: 22 is mutated to "T", and the mutation "3'UTR.76_77insT" correspond to the insertion of nucleotide "T" between the positions 2879 and 2880 of SEQ ID NO: 22.

Herein, where a base is mentioned, G refers to guanine, T refers to thymine, A refers to adenine, C refers to cytosine, and U refers to uracil.

As used herein, the "non-mutated CPR-b gene" refers to a CPR-b gene that does not comprises the mutation -322G>A, 3'UTR.19C>T or 3'UTR.76_77insT according to the invention, e.g. a naturally occurring wild type allele, such as the CPR-b gene with the Accession Number AY823228 in the GenBank. An example of non-mutated CPR-b gene is set forth in SEQ ID NO: 22. The non-mutated CPR-b gene may contain other mutations, such as a silent mutation in the coding region which does not result in the alteration of the encoded amino acid.

As used herein, "non-mutant microorganism" refers to a microorganism which does not contain the mutated CPR-b gene or homologous gene thereof according to the invention, e.g. contain only the CPR-b gene with the Accession Number AY823228 in the GenBank. In an embodiment, the non-mutant microorganism contains a non-mutated CPR-b gene according to the invention.

The invention screens out a strain having a mutated CPR-b gene, relative to GenBank Accession Number AY823228, taking the first base upstream of the start codon ATG as -1, which comprises a base mutation -322 G>A in its promoter region, and taking the first base downstream of the stop codon TAG as 1, comprises base mutations 3'UTR.19C>T and 3'UTR.76_77insT in its terminator region.

Homologous genes refer to two or more gene sequences with at least 80% similarity, including orthologous genes, paralogous genes and/or xenologous genes. The homologous gene of the CPR-b gene in the invention refers to either the orthologous gene of the CPR-b gene, or paralogous gene or xenologous gene of the CPR-b gene.

Sequence identity refers to the percent identity of the residues of a polynucleotide sequence variant with a non-variant sequence after sequence alignment and introduction of gaps. In some embodiments, the polynucleotide variant has at least about 70%, at least about 75%, at least about 80%, at least about 90%, at least about 95%, at least about 96%, at least about 97%, at least about 98%, at least about 99%, at least about 99.1%, at least about 99.2%, at least about 99.3%, 99.4%, at least about 99.5%, at least about 99.6%, 99.7%, at least about 99.8%, at least about 99.9%, at least about 99.91%, at least about 99.92%, at least about 99.93%, at least about 99.94%, at least about 99.95%, or at least about 99.96% polynucleotide homology with the polynucleotide described herein.

As used herein, the terms "homology" and "identity" are used interchangeably herein to refer to the extent of non-variance of nucleotide sequences, which can be detected through the number of identical nucleotide bases by aligning a polynucleotide with a reference polynucleotide. The sequence identity can be determined by standard alignment algorithm programs used with default gap penalties established by each supplier. Homologous nucleic acid molecules refer to a pre-determined number of identical or homologous nucleotides. Homology includes substitutions that do not change the encoded amino acid ("silent substitution") as well as identical residues. Substantially homologous nucleic acid molecules hybridize typically at moderate stringency or high stringency all along the length of the nucleic acid or along at least about 70%, 80% or 90% of the full-length nucleic acid molecule of interest. Nucleic acid molecules that contain degenerate codons in place of codons in the hybridizing nucleic acid molecule are also contemplated in the invention. Whether any two nucleic acid molecules have nucleotide sequences that are at least 80%, 85%, 90%, 95%, 96%, 97%, 98% or 99% "identical" can be determined using a known computer algorithm such as the BLASTN, FASTA, DNAStar and Gap (University of Wisconsin Genetics Computer Group (UWG), Madison WI, USA). Percent homology or identity of nucleic acid molecules can be determined, e.g. by comparing sequence information using a GAP computer program (e.g., Needleman et al. J. Mol. Biol. 48: 443 (1970), as revised by Smith and Waterman (Adv. Appl. Math. 2: 482 (1981)). Briefly, a GAP program defines similarity as the number of aligned symbols (i.e., nucleotides) which are similar, divided by the total number of symbols in the shorter of the two sequences.

Directed evolution refers to a process of simulating a natural selection by technology means. Through an artificially created mutation and specific screening pressure, a protein or nucleic acid is mutated in a specific direction, thereby realizing an evolutionary process in nature that requires thousands of years to complete in a short period of time at the molecular level. The methods for performing directed evolution are known in the art, e.g. error-prone PCR (e.g. Technique, 1, 11-15, 1989; Genome Research, 2, 28-33, 1992).

In some embodiments, in the error-prone PCR of the invention, the concentration of Mg²⁺ is in a range of 1 to 10 mM, preferably 2 to 8 mM, more preferably 5 to 6 mM, and/or the concentration of dNTP is from 0.1 to 5mM, preferably from 0.2 to 3mM, more preferably 0.5 to 2mM, and more preferably from 0.8 to 1.5 mM, for example 1 mM, and/or addition of freshly prepared MnCl₂ to a final concentration of 0.1 to 5mM, preferably 0.2 to 2 mM, more preferably 0.3 to 1 mM, and more preferably 0.4 to 0.7 mM, such as 0.5 mM. In some embodiments, the rate of mutation is increased by decreasing the amount of template and appropriately increasing PCR cycles to 40 or more, e.g. 41, 42, 43, 44, 45, 46, 47, 48, 49, 50, 55, 60 or more PCR cycles.

PCR overlap extension, also known as SOE (gene splicing by overlap extension) PCR, refers to a method of splicing different DNA fragments together via PCR amplification by designing primers having complementary ends.

Homologous recombination refers to the recombination between DNA molecules that relies on sequence similarity, most commonly found within cells to repair mutations that occur during mitosis. Homologous recombination technology has been widely used in genome editing, including gene knockout, gene repair and introduction of a new gene to a specific site. A class of microorganisms represented by *Saccharomyces cerevisiae* has a very high rate of homologous recombination within cells which does not depend on sequence specificity and is obviously advantageous in genome editing. Site-specific recombination relies on the participation of specific sites and site-specific recombinases, and the recombination occurs only between specific sites, such as Cre/loxP, FLP/FRT, and the like. The homologous recombination technology used in the invention does not belong to site-specific recombination, and recombination relies on the intracellular DNA repair system.

The resistance marker refers to a type of selective markers that often has the ability of conferring a transformant survival in the presence of an antibiotic. The resistance marker gene includes NPT, HPT, HYG, BLA and CAT, etc., which are resistant to kanamycin, hygromycin, ampicillin/carbenicillin, and chloramphenicol, respectively. Preferably, the resistance marker gene is the hygromycin B resistance gene HYG.

During fermentation, the fermentation medium comprises a carbon source, a nitrogen source, an inorganic salt and a nutritional factor.

In some embodiments, the carbon source comprises one or more selected from the group consisting of glucose, sucrose and maltose; and/or the carbon source is added in an amount of 1% to10% (w/v), such as 1.5%, 2.0%, 2.5%, 3.0%, 3.5%, 4.0%, 4.5%, 5.0%, 6.0%, 7.0%, 8.0%, or 9.0%.

In some embodiments, the nitrogen source comprises one or more selected from the group consisting of peptone, yeast extract, corn syrup, ammonium sulfate, urea, and potassium nitrate; and/or the nitrogen source is added in a total amount of 0.1%-3% (w/v), such as 0.2%, 0.4%, 0.5%, 0.6%, 0.8%, 1.0%, 1.2%, 1.5%, 1.8%, 2.0%, or 2.5%.

In some embodiments, the inorganic salt comprises one or more selected from the group consisting of potassium dihydrogen phosphate, potassium chloride, magnesium sulfate, calcium chloride, ferric chloride, and copper sulfate; and/or the inorganic salt is added in a total amount of 0.1%-1.5% (w/v), such as 0.2%, 0.3%, 0.4%, 0.5%, 0.6%, 0.7%, 0.8%, 0.9%, 1.0%, 1.1%, 1.2%, 1.3%, or 1.4%.

In some embodiments, the nutritional factor comprises one or more selected from the group consisting of vitamin B1, vitamin B2, vitamin C, and biotin; and/or the nutritional factor is added in a total amount of 0-1% (w /v), such as 0.2%, 0.3%, 0.4%, 0.5%, 0.6%, 0.7%, 0.8%, or 0.9%. According to common knowledge in the art of fermentation, the percentage in the invention is the mass to volume ratio, i.e. w/v; and % indicates g/100 mL.

Those skilled in the art can easily determine the amount of the above substances to be added.

In one embodiment of the invention, the inoculation amount of the strain for fermentation is 10% to 30%, for example 11%, 12%, 13%, 14%, 15%, 16%, 17%, 18%, 19%, 20%, 22%, 24%, 25%, 27%, or 29%. When the strain is cultured to an optical density (OD₆₂₀) of 0.5 or more (diluted 30 folds), the substrate is added for fermentation conversion.

Extraction and purification of a long-chain dibasic acid: the fermentation broth obtained by fermentation is subjected to extraction and purification treatment to obtain a long-chain dibasic acid product. The step of extraction and purification comprises: sterilization and acidification of the fermentation broth, as well as acidification, solid-liquid separation, and/or solvent crystallization of the obtained clear liquid.

The extraction and purification according to the present invention may be repeated more than once, and performing multiple extraction and purification steps contribute to further reducing the impurity content in the dibasic acid product. For example, in one embodiment of the present invention, with reference to the refining process in Example 1 of Chinese Patent Application CN 101985416 A, C12 long-chain dibasic acid product obtained by the present invention is further treated, and the content of lauric acid impurity in the obtained C12 long-chain dibasic acid can be decreased from greater than 5000 ppm before treatment to less than 4,000 ppm, for example, less than 3000 ppm, less than 2000 ppm, less than 1000 ppm, less than 500 ppm, less than 400 ppm, less than 300 ppm, or even less than 250 ppm, 200 ppm or 150 ppm.

The fermentation broth includes a fermentation broth containing a salt of the long-chain dibasic acid produced during the biological fermentation for producing the long-chain dibasic acid. The fermentation broth containing a salt of a long-chain dibasic acid may contain sodium salt, potassium salt or ammonium salt of the long-chain dibasic acid.

The sterilization is preferably membrane filtration: the residual bacteria and large proteins are separated by using a filtration membrane, and are effectively separated from the fermentation broth containing the salt of the long-chain dibasic acid. Further, the ceramic membrane filtration process is preferred. When membrane filtration is carried out using a ceramic membrane, it is preferred that the pre-membrane pressure is 0.2 to 0.4 MPa; preferably, the pore size of the filtration membrane is 0.05 to 0.2 µm.

The acidification is a treatment of acidifying the obtained membrane supernatant containing a salt of a long-chain dibasic acid after membrane filtration, and the salt of the long-chain dibasic acid is converted into a long-chain dibasic acid precipitate by adding an acid. It is preferred to use an inorganic acid such as sulfuric acid, hydrochloric acid, nitric acid, or mixture thereof for acidification. The inorganic acid during the acidification treatment is added in an amount sufficient to precipitate the long-chain dibasic acid in the solution, mainly based on the endpoint pH of the solution, preferably the acidification end point pH is lower than 5, and more preferably lower than 4.0. When an inorganic acid is added for acidification, the long-chain dibasic acid precipitate and corresponding inorganic salt solution can be obtained.

The solid-liquid separation is to separate the obtained long-chain dibasic acid precipitate from the acidified mother liquid, and the solid-liquid separation includes filtration or/and centrifugation, and a commonly used solid-liquid separation device can be used.

Preferably, the step of extraction and purification further comprises decolorization of the fermentation broth containing a long-chain dibasic acid salt, adding activated carbon to the fermentation broth or the membrane supernatant containing the salt of the long-chain dibasic acid for decolorization treatment, and removing the activated carbon by filtration after decolorization treatment. Decolorization step can further remove impurities in the long-chain dibasic acid solution. Preferably, the amount of activated carbon added is 0.1-5 wt%, preferably 1-3 wt % (relative to the amount of the long-chain dibasic acid contained in the solution).

The solvent crystallization, i.e., dissolving a long-chain dibasic acid precipitate in an organic solvent, and crystallizing the long-chain dibasic acid by cooling, evaporation, and separating-out, and isolating the crystal to obtain a purified long-chain dibasic acid. The organic solvent comprises one or more of alcohol, acid, ketone and ester; wherein the alcohol comprises one or more of methanol, ethanol, isopropanol, n-propanol and *n-*butanol; the acid comprises acetic acid; the ketone comprises acetone; and the ester comprises ethyl acetate and/or butyl acetate.

In another preferred embodiment, the long-chain dibasic acid precipitate is dissolved in an organic solvent, and then decolorized, and then separated to obtain a clear solution. When decolorized with activated carbon, the decolorization temperature is 85 to 100°C, and the decolorization time is 15 to 165 min. In another preferred embodiment, after separating the clear liquid, cooling and crystallizing is carried out, and cooling and crystallizing may include the steps of: first cooling to 65-80°C, incubating for 1 to 2 hours, then cooling to 25-35°C, and crystallizing. In another preferred embodiment, after crystallization, the resulting crystal is separated, thereby obtaining the long-chain dibasic acid, and the crystal may be separated by centrifugation.

In some embodiments, the present invention relates to the production of nylon filaments, engineering plastics, synthetic fragrances, cold-resistant plasticizers, advanced lubricating oils, and polyamide hot melt adhesives using the dibasic acid products obtained above.

As used herein, "optional" or "optionally" means that the subsequently described event or circumstance does or does not occur, and that the description comprises instances where the event or circumstance occurs or does not occur. For example, "optionally a step" means that the step is present or not present.

As used herein, the term "about" means a range of values including the specified value, which a person of ordinary skill in the art would consider reasonably similar to the specified value. In some embodiments, the term "about" means within a standard deviation using measurements generally acceptable in the art. In some embodiments, "about" means a range extending to +/- 10% of the specified value.

The invention will be further illustrated by the following non-limiting examples. Those skilled in the art will recognize that modifications can be made to the invention without departing from the spirit thereof, and such modifications also fall within the scope of the invention.

The following experimental methods are all conventional methods unless otherwise specified, and the experimental materials used can be easily obtained from commercial companies unless otherwise specified.

### Example 1 Culture medium, culture and fermentation methods and the method of detecting dibasic acid

1. The formulation of YPD medium (w/v): 2% peptone, 2% glucose and 1% yeast extract (OXOID, LP0021). 1.5-2% agar powder was added to form a solid medium.
   During culturing, a single colony was picked into a 2 mL centrifuge tube containing 1mL YPD liquid medium, incubated at 30°C on a shaker at 250 RPM for 1 day.
2. The formulation of seed medium (w/v): sucrose 10-20 g/L (specifically used, 10 g/L), yeast extract 3-8 g/L (specifically used, 3 g/L), industrial fermentation corn syrup (corn syrup for short, with total nitrogen content of 2.5 wt%) 2-4 g/L (specifically used, 2 g/L), KH₂PO₄ 4-12 g/L (specifically used, 4 g/L), urea 0.5-4 g/L (specifically used, 0.5 g/L) (separately sterilized at 115°C for 20 min), and the fermentation substrate was *n-*dodecane, n-decane, and n-hexadecane, at 20 mL/L, respectively.
   During culturing, the inoculum obtained in step 1 was inoculated into a 500mL shake flask containing 30mL seed medium. The amount of inoculum was 3-5% and incubated at 30°C on a shaker at 250 RPM until OD₆₂₀ reached 0.8 (by 30-fold dilution).
3. Fermentation medium(w/v): sucrose 10-40 g/L (specifically used, 10 g/L), corn syrup (total nitrogen content of 2.5 wt%) 1-5 g/L (specifically used, 1 g/L), yeast extract 4-12 g/L (specifically used, 4 g/L), NaCl 0-3g/L (not used), KNO₃ 4-12g/L (specifically used, 4 g/L), KH₂PO₄ 4-12 g/L (specifically used, 4 g/L), urea 0.5-3 g/L (specifically used, 0.5 g/L) (separately sterilized at 115°C for 20 min), and the fermentation substrate was n-dodecane, n-decane, and n-hexadecane, at 300-400mL/L (specifically used, 300mL/L), respectively, and acrylic acid 4g/L, and 1N HCl and 1N NaOH were used to adjust pH to 7.5-7.6.
   During culturing, the seed solution obtained in step 2 was inoculated into a 500mL shake flask containing 15mL fermentation medium. The amount of inoculum was 10-30% and incubated at 30°C on a shaker at 250 RPM for 90-144h. During culturing, the pH value was adjusted to the specified range by adding acid or base at a certain interval of time.
4. Steps for determining the yield of dibasic acid and the content of fatty acid impurity by gas chromatography (GC)

(1) Detection of fermentation broth product and impurity content: The fermentation broth was pretreated by conventional gas chromatography and detected by gas chromatography. The chromatographic conditions were as follows:
   Column: Supelco SPB-50 30m ^{∗} 0.53mm ^{∗} 0.5 µm (Cat. No. 54983).
   Gas Chromatograph (Shimadzu, GC-2014).
   Method: The initial temperature was 100°C, and the temperature was raised to 230°C at a rate of 15°C/min and kept for 2 min. The carrier gas was hydrogen, the inlet temperature was 280°C, the FID temperature was 280°C, and the injection volume was 4µL.
   The yield of the dibasic acid was calculated based on the ratio of the peak area of the dibasic acid product and the internal standard peak area with a known concentration, and the impurity content was calculated by the peak area of the dibasic acid product to the peak area of the impurity.
(2) Determination of purity and impurity content of solid product: the solid product was pretreated by conventional gas chromatography and detected by gas chromatography,
   Chromatographic conditions: Column: Supelco SPB-50 30 m ^{∗} 0.53 mm ^{∗} 0.5 µm (Cat. No. 54583).

Gas Chromatograph (Shimadzu, GC-2014).

Method: The initial temperature was 100°C, and the temperature was raised to 230°C at a rate of 15°C/min and kept for 2 min. The carrier gas was hydrogen, the inlet temperature was 280°C, the FID temperature was 280°C, and the injection volume was 4µL.

The purity of the product and impurity content were calculated from the peak area of the dibasic acid product and the peak area of impurity.

### Example 2 Preparation of CPR-b Mutation Template

1. The genomic DNA of *Candida tropicalis* CCTCC M2011192 was extracted by using Ezup Yeast Genomic DNA Extraction Kit (Sangon, Cat No. 518257). The method with liquid nitrogen grinding was used in favor of increasing the cell wall disruption efficiency. Genomic DNA obtained by this method was used as template for error-prone PCR. The obtained mutation-free product was called CPR-b and was confirmed by sequencing to be identical to the sequence set forth by GenBank Accession Number: AY823228.

### 2. Error-prone PCR

The concentration of Mg²⁺ was adjusted (2-8 mM, increasing by 0.5mM) and the CPR-b gene was amplified by error-prone PCR using normal Taq enzyme (Takara, Cat No. R001B). The primers were as follows:
CPR-b-F: 5'-CAAAACAGCACTCCGCTTGT-3' (SEQ ID NO.1)
CPR-b-R: 5'-GGATGACGTGTGTGGCTTGA-3'(SEQ ID NO.2)

PCR reaction conditions were:
Step 1: 98°C for 30 s,
Step 2: 98°C for 10 s, 55°C for 30 s, 72°C for 3 m, 35 cycles,
Step 3: 72°C for 5 m

The PCR product was subjected to electrophoresis on 1% agarose gel, then recovered and purified by using the Axygen Gel Recovery Kit (Axygen, AP-GX-250G).

### Example 3 Preparation of Homologous Recombination Template

All DNA fragments in this example were obtained by amplification using PrimeSTAR® HS High Fidelity DNA polymerase (Takara, R040A). The DNA fragments were subjected to 1% agarose gel electrophoresis, followed by recovery and purification by using the Axygen Gel Recovery Kit.
(1) Amplification of the upstream and downstream homologous recombination fragments. The template was the above genomic DNA of *Candida tropicalis.* The primer sequences were as follows:
   CPR-b_Upstream-F: 5'-TTTGCGCGAGTAACATGTGC-3' (SEQ ID NO.3)
   CPR-b_Upstream-R: 5'-AATGATTCCTGCGAGGGGTG-3' (SEQ ID NO.4)
      The PCR reaction conditions were as follows:
      Step 1: 98°C for 30 s,
      Step 2: 98°C for 10 s, 55°C for 10 s, 72°C for 25 s, 30 cycles,
      Step 3: 72°C for 5 m.
   CPR-b_Downstream-F: 5'-TTTAGTACAGTATCTCCAATCC-3' (SEQ ID NO.5)
   CPR-b_Downstream-R: 5'-ACGTCTATATTGTGGATGGC-3' (SEQ ID NO.6)
      The PCR reaction conditions were as follows:
      Step 1: 98°C for 30 s,
      Step 2: 98°C for 10 s, 48°C for 10 s, 72°C for 25 s, 30 cycles,
      Step 3: 72°C for 5 m.
      The resultant products were designated as CPR-b_Upstream and CPR-b_Downstream, respectively, and verified by sequencing, wherein the sequences thereof were set forth in SEQ ID NOS. 14 and 15.
(2) Amplification of the resistance marker (HYG, hygromycin resistance gene). The amplification template was the vector pCIB2 (SEQ ID NO.16) owned by our company. The primer sequences were as follows:
   CPR_HYG-F: 5'-CCGTTGGTAATGCCGGGATAGCATGCGAACCCGAAAATGG-3' (SEQ ID NO.7) CPR_HYG-R:
   5'-GGATTGGAGATACTGTACTAAAGCTAGCAGCTGGATTTCACT-3' (SEQ ID NO.8).
   The PCR reaction conditions were as follows:
   Step 1: 98°C for 30 s,
   Step 2: 98°C for 10 s, 55°C for 10 s, 72°C for 1 m 50 s, 5 cycles,
   Step 3: 98°C for 10 s, 72°C for 2 m, 25 cycles,
   Step 4: 72°C for 5 m.
   The resultant product, called HYG, was verified by sequencing, as set forth in SEQ ID NO. 9.
(3) PCR overlap extension to obtain a complete recombination template.

The four PCR fragments recovered above were subjected to overlap extension to obtain a homologous recombination template, which was recovered and purified. The specific method was as follows:
Overlap extension PCR was performed by adding an equimolar amount of the fragments CPR-b_Upstream, CPR-b, HYG and CPR-b_Downstream as templates, with primers CPR-b_Upstream-F and CPR-b_Downstream-R, and using PrimeSTAR® HS High Fidelity DNA polymerase.

The PCR reaction conditions were as follows:
Step 1: 98°C for 30 s,
Step 2: 98°C for 10 s, 50°C for 10 s, 72°C for 5 m 30 s, 30 cycles,
Step 3: 72°C for 8 m.

The recombination fragment with a size of approximately 5.1Kb was recovered and purified after gel electrophoresis.

Figure 1 is a schematic diagram of the integration of the CPR-b gene with a mutation site by the homologous recombination and removal of the hygromycin resistance marker according to the present invention.

### Example 4 Construction of Candida tropicalis CPR-b Gene Mutant Library

### 1. Preparation of Yeast Electroporation-competent Cells

The yeast cells *Candida tropicalis* CCTCC M2011192 subjected to overnight incubation at 30°C on a shaker at 250 RPM were inoculated into 100mL of the YPD medium of Example 1 to OD₆₂₀ of 0.1, and cultured under the same conditions to OD₆₂₀ of 1.3. The cells were collected by centrifugation at 3000g, 4°C. Cells were washed twice with ice-cold sterile water and collected, and then the cells were re-suspended in 10mL of ice-cold 1M sorbitol solution. The cells were collected by centrifugation at 4°C, 1500g and re-suspended in 1mL sorbitol solution above mentioned. Aliquots of 100µL of cell suspension were for genetic transformation.

### 2. Competent Yeast Cell Electroporation

1µg of the DNA fragments for recombination recovered in step (3) of Example 3 were added to the above competent cells, and placed on ice for 5 min and transferred to a 0.2cm cuvette, and then performing electroporation (BioRad, Micropulser™ Electroporator, program SC2). Afterwards, a mixture of 1mL of YPD and 1M sorbitol (1:1, v/v) was immediately added, and cultured at 30°C, 200 RPM for 2 hours. The bacterial cells were collected and plated on a YPD medium plate with 100mg/L of hygromycin B, placed still at 30°C for 2-3 days until single colonies appeared.

### Example 5 Screening of Mutant Strains

1. Screening method: single colonies obtained in Example 4 were picked into a 2mL centrifuge tube with 1mL YPD medium of Example 1 (containing 100mg/L hygromycin B), and cultured at 30°C on a shaker at 250 RPM for 1 day. The above bacterial solution was inoculated into a 500-mL shake flask with 30mL of the seed medium of Example 1 (containing 100mg/L hygromycin B). The inoculum amount was 3%, cultured at 250 RPM and 30°C until OD₆₂₀ reached 0.8 (30-fold dilution). The seed solution was inoculated into a 500-mL shake flask containing 15mL of the fermentation medium of Example 1, the inoculum amount was 20%, and the substrate was n-dodecane in the fermentation medium. The culture at 250 RPM and 30°C was continued until the end of the fermentation.
The strain CCTCC M2011192 was used as control: the medium, culture and fermentation methods were the same as above except that the medium did not contain hygromycin B.
0.5g sample of the above fermentation broth was taken respectively and subjected to GC assay using the method described in Example 1 (4), and the content of C12 dibasic acid content and the mass ratio of lauric acid impurity were calculated, and the results are shown in Table 1 below.
2. Screening results: a candidate strain with a significant reduction in lauric acid impurity content compared to the original strain CCTCC M2011192 was screened out, designated as 5473HYG.

**Table1**

| Strain | Control CCTCC M2011192 | 5473HYG |
|---|---|---|
| Yield of C12 dibasic acid (mg/g) | 150.8 | 151.6 |
| Mass ratio of lauric acid impurity (%) | 1.02 | 0.64 |

The mass ratio of lauric acid impurity of the present invention was the mass percentage of it to C12 dibasic acid. From Table 1, the mass ratio of lauric acid impurity was decreased by 37.3%.

### Example 6 Sequence Analysis of CPR-b Gene in the Mutant Strain

1. According to the method of Example 2, the genomic DNAs of the yeast CCTCC M2011192 and 5473HYG were extracted, and the CPR-b gene was amplified using PrimeSTAR® HS High Fidelity DNA polymerase (Takara), using the primers CPR-b-F and CPR-b-R. The PCR reaction conditions were as follows:
   The PCR reaction conditions were:
   Step 1: 98°C for 30 s,
   Step 2: 98°C for 10 s, 50°C for 10 s, 72°C for 3 m, 30 cycles,
   Step 3: 72°C for 5 m.
2. After completion of the PCR, the product was subject to gel electrophoresis and recovered and purified.
3. Addition of As to the purified PCR fragment: 20µL of recovered PCR amplified fragment was added to 4µL of 10X Takara Taq Buffer, 3.2µL of dNTP (each 10mM) and 0.2µL of Takara Taq, supplemented with ddH₂O to 40µL, incubated at 72°C for 20 minutes, and recovered by Axygen PCR purification kit.
4. TA cloning. 4µL of the PCR fragment recovered after addition of As were added to 1µL pMD19-T vector backbone and 5µL Solution I, mixed well and incubated at 16°C for 30 min. The ligation product was transformed into DH5α chemical competent cells and positive clones were picked and sent to Majorbio for sequencing.

The results showed that: the sequence of the CPR-b gene of the parental CCTCC M2011192 was identical to the sequence in the GenBANK database (Accession Number: AY823228), while the mutant strain 5473HYG had base mutations in the promoter region and the terminator region. As shown in Figure 2, one base mutation -322G>A occurred in the promoter region (indicated with black box in the sequence alignment result); and base mutations 3'UTR.19C>T and 3'UTR.76_77insT occurred in its terminator region, taking the first base downstream of the stop codon TAG as 1. The sequence was as set forth in SEQ ID NO: 13.

### Example 7 Removal of the resistance marker

### 1. Preparation of homologous recombination template CPR-b-2

The *Candida tropicalis* ATCC26336 genomic DNA was used as template to amplify CPR-b-2, and recovered after gel electrophoresis. The sequence obtained was verified by sequencing and shown in SEQ ID NO. 12. The primer sequences and PCR reaction conditions were as follows:
CPR-b-2F: 5'-ATTACGAAACATAGGTCAACT-3' (SEQ ID NO.10)
CPR-b-2R: 5'-TAACCATATCCATACGTCGC-3' (SEQ ID NO.11)
   Step 1: 98°C for 30 s,
   Step 2: 98°C for 10 s, 50°C for 10 s, 72°C for 40 s, 30 cycles,
   Step 3: 72°C for 5 m.

### 2. Removal of the resistance marker

Freshly electrocompetent cells of the strain 5473HYG were prepared and 1µg of recovered CPR-b-2 was added thereto. After being placed on ice for 5 min, the cells were quickly transferred to a pre-chilled 0.2cm cuvette on ice and transformed by electroporation (supra, 1.5kV, 25 uFD, 200 ohms). A mixture of 1mL YPD and 1M sorbitol (1:1, v/v) was quickly added, and incubated at 30°C and 200 RPM for 2 hours. The bacterial cells were collected and plated on an YPD medium plate without an antibiotic, and cultured at 30°C for 2-3 days until single colonies appeared.

### 3. Screening strains with the resistance marker removed

Single colonies were picked and correspondingly inoculated on YPD plates with and without hygromycin (100 mg/L). Single colonies that could grow on the medium without the antibiotic but could not grow on the medium with the antibiotic were picked and inoculated to a 2mL centrifuge tube containing 1mL of the YPD medium, incubated overnight at 4°C and 250 RPM, and the colony PCR was used to determine whether the resistance marker was removed or not in the next day. The DNA polymerase used was Takara Taq, with the primers:
a) CPR-b-2F & CPR-b-2R , PCR reaction conditions were the same as above;
b) HYG-F: 5'-CTCGGAGGGCGAAGAATCTC-3' (SEQ ID NO.17) HYG-R: 5'-CAATGACCGCTGTTATGCGG-3' (SEQ ID NO.18).

The PCR conditions were as follows:
Step 1: 98°C for 30 s,
Step 2: 98°C for 10 s, 50°C for 30 s, 72°C for 35 s, 30 cycles,
Step 3: 72°C for 5 min.

### 4. Screening results

By colony PCR, one strain with the resistance marker removed was screened out, and confirmed by sequencing that this strain has one base mutation -322G>A in the promoter region of the CPR-b gene, and taking the first base downstream of the stop codon TAG as 1, base mutations 3'UTR.19C>T and 3'UTR.76_77insT in the terminator region of the CPR-b gene. The hygromycin resistance marker gene was removed. The strain was eventually designated as 5473.

### Example 8 Fermentation Production of a Long-chain dibasic acid by Strain 5473

Fermentation: Strain 5473 was inoculated to a 2mL centrifuge tube containing 1 mL of YPD medium of Example 1, and incubated at 30°C on a shaker at 250 RPM for 1 day. The above bacterial solution was inoculated into a 500-mL shake flask with 30 mL of the seed medium of Example 1, wherein the inoculation amount was 3%, and cultured at 250 RPM and 30°C on a shaker until OD₆₂₀ reached 0.8 (after 30-fold dilution). The seed solution was inoculated into a shake flask containing 15 mL of the fermentation medium of Example 1, wherein the inoculation amount was 20%, and the substrate was *n-*dodecane in the fermentation medium. The culturing on shaker at 250 RPM and 30°C was continued until the completion of the fermentation. The strain CCTCC M2011192 was used as control, and the medium, culture and fermentation methods were the same as described above.

A 0.5 g sample of the above fermentation broth was taken and measured using the method described in Example 1 (4), and the production of C12 dibasic acid and the mass ratio of lauric acid impurity were calculated, as shown in Table 2 below.

**Table 2**

| Strain | CCTCC M2011192 | 5473 |
|---|---|---|
| Yield of C12 dibasic acid (mg/g) | 152.4 | 153.7 |
| Mass ratio of lauric acid impurity (%) | 1.11 | 0.66 |

It can be seen from Table 2 that the mass ratio of lauric acid impurity was decreased by 40.5% after removal of the resistance marker.

Extraction and purification:
(1) The pH of the above fermentation broth was adjusted to 8.5 with 30% (mass concentration) sodium hydroxide solution, the concentration of long-chain dibasic acid was adjusted by adding water to 8.9 wt% and heated to 45°C, and the fermentation broth was filtered with a ceramic membrane with pore size of 0.05µm (purchased from Suntar Membrane Technology (Xiamen) Co., Ltd.). The area of the ceramic membrane used was 0.84 square meters, and the membrane pressure was set to 0.3 MPa. The membrane supernatant was collected.
(2) The obtained membrane supernatant was decolorized by adding 5 wt% of powdered activated carbon (relative to the amount of long-chain dibasic acid contained in the solution) at 60°C, and filtered to obtain a clear liquid.
(3) The clear liquid was further added with sulfuric acid, the pH was adjusted to 3.2, cooled to 30°C, and filtered to obtain a wet solid. The filter cake was washed with pure water the weight of which was 3 times to the wet solid, and filtered and dried to obtain the primary C12 dibasic acid product.
(4) Acetic acid at a concentration of 97% whose amount was 3.5 times relative to the weight of the primary dibasic acid product was added to the primary C12 dibasic acid product and heated to 85°C to dissolve, and 1% macroporous powdered activated carbon (relative to the weight of the primary dibasic acid product) was added for decolorization and kept at 85°C for 1 hour, and hot-filtered to obtain a clear liquid. The temperature of the solution was decreased at a rate of 10°C/hour to obtain a long-chain dibasic acid crystal solution at 30°C. The solution was filtered and the solvent of the wet solid was washed with water, and dried to obtain C12 dibasic acid secondary product.

The purity of C12 dibasic acid and the content of lauric acid impurity were determined and calculated using the method described in Example 1 (4), as shown in Table 3 below:

**Table 3**

| | Strain | CCTCC M2011192 | 5473 |
|---|---|---|---|
| The primary C12 dibasic acid product | Purity of C12 dibasic acid (%) | 97.41 | 98.35 |
| | Content of lauric acid impurity (ppm) | 5500 | 2600 |
| C12 dibasic acid secondary product | Purity of C12 dibasic acid (%) | 99.41 | 99.8 |
| | Content of lauric acid impurity (ppm) | 350 | 156 |

**Example 9** To further verify the above mutations, the genomic DNA of the yeast 5473HYG was extracted, and the DNA fragment containing the mutated CPR-b and HYG resistance genes was amplified via PCR using the PrimeSTAR® HS high-fidelity DNA polymerase, and subjected to gel electrophoresis, and recovered and purified, with a size of approximately 4.7Kb, and verified by sequencing, the sequence of which was set forth in SEQ ID NO.19.
CPR-3-F: 5'-GGGATCTCCTCCGCAGTTTA-3' (SEQ ID NO. 20)
CPR-3-R: 5'-ATTGTGGATGGCCAGAAGTT-3' (SEQ ID NO. 21)

The PCR reaction conditions were:
Step 1: 98°C 30 s
Step 2: 98°C 10 s, 53°C 30 s, 72°C 5 m, 30 cycles
Step 3: 72°C 5 m

The process of introducing via homologous recombination the above DNA fragment (SEQ ID NO. 19) into the strain CCTCC M2011192 was the same as in Example 4, and the sequencing procedure of the CPR-b gene of the single clone obtained by screening was the same as in Example 6. It was verified by sequencing that the selected single clone was integrated with the CPR-b gene with mutations, and the mutation sites were consistent with SEQ ID NO.13. One of the bacterial strains was named as 5474HYG.

The fermentation method was the same as described in Example 5, and the strains used were CCTCC M2011192, 5473HYG and 5474HYG. After the fermentation, the samples of 0.5g of the above fermentation broths were taken to calculate the yield of the dibasic acid and the content of lauric acid impurity, as shown in Table 4. The results showed that, consistent with 5473HYG, the content of lauric acid impurity in 5474HYG was significantly decreased compared to that of the control CCTCC M2011192.

**Table 4**

| Strain | CCTCC M2011192 | 5473HYG | 5474HYG |
|---|---|---|---|
| Yield of C12 dibasic acid (mg/g) | 151.2 | 152.5 | 152.3 |
| Mass ratio of lauric acid impurity (%) | 1.01 | 0.67 | 0.67 |

### Example 10 Production of long-chain C10 dibasic acid by Fermentation of strain 5473

Fermentation: Strain 5473 was inoculated to a 2 mL centrifuge tube containing 1 mL of YPD medium of Example 1, incubated at 30°C on a shaker at 250 RPM for 1 day. The above bacterial solution was inoculated into a 500-mL shake flask with 30 mL of the seed medium of Example1, wherein the inoculation amount was 3%, and cultured at 250 RPM and 30°C for 36-48h until OD₆₂₀ reached 0.8 (after 30-fold dilution). The seed solution was inoculated into a shake flask containing 15mL of the fermentation medium of Example 1, wherein the inoculation amount was 20%, and the substrate was *n-*decane in the fermentation medium. The culture on a shaker at 250 RPM and 30°C was continued until the end of the fermentation. The strain CCTCC M2011192 was used as control, and the medium, culture and fermentation methods were the same as described above.

A 0.5 g sample of the above fermentation broth was taken and measured using the method described in Example 1 (4), and the yield of C10 dibasic acid and the mass ratio of fatty acid capric acid impurity were calculated, as shown in Table 5 below.

**Table 5**

| Strain | CCTCC M2011192 | 5473 |
|---|---|---|
| Yield of C10 dibasic acid (mg/g) | 120.9 | 123.4 |
| Mass ratio of capric acid impurity (%) | 0.72 | 0.42 |

It can be seen from Table 5 that the mass ratio of capric acid impurity was decreased by 41.7%.

Extraction and purification steps: they were the same as the extraction and purification steps of Example 8. The purity of the primary and secondary C10 dibasic acid product and the content of capric acid impurity were determined and calculated using the method described in Example 1 (4), as shown in Table 6 below:

**Table 6**

| C10 dibasic acid | Strain | CCTCC M2011192 | 5473 |
|---|---|---|---|
| Primary product | Purity of C10 dibasic acid (%) | 98.05 | 98.90 |
| | Content of capric acid impurity (ppm) | 2380 | 1570 |
| Secondary product | Purity of C10 dibasic acid (%) | 99.12 | 99.87 |
| | Content of capric acid impurity (ppm) | 210 | 105 |

### Example 11 Production of C16 long-chain dibasic acid by Fermentation of the strain 5473

Fermentation: Strain 5473 was inoculated to a 2 mL centrifuge tube containing 1 mL of YPD medium of Example 1, and incubated at 30°C on a shaker at 250 RPM for 1 day. The above bacterial solution was inoculated into a 500-mL shake flask with 30 mL of the seed medium of Example 1, wherein the inoculation amount was 3%, and cultured at 250 RPM and 30°C for 36-48h until OD₆₂₀ reached 0.8 (after 30-fold dilution). The seed solution was inoculated into a shake flask containing 15 mL of the fermentation medium of Example 1, wherein the inoculation amount was 20%, and the substrate was *n-*hexadecane in the fermentation medium. The culture on a shaker at 250 RPM and 30°C was continued until the end of the fermentation. The strain CCTCC M2011192 was used as control, and the medium, culture and fermentation methods were the same as described above.

A 0.5 g sample of the above fermentation broth was taken, and the yield of C16 dibasic acid and the mass ratio of fatty acid palmitic acid impurity were measured using the method described in Example 1 (4), and the results were shown in Table 7 below.

**Table 7**

| Strain | CCTCC M2011192 | 5473 |
|---|---|---|
| Yield of C16 dibasic acid (mg/g) | 122.9 | 125.8 |
| Mass ratio of palmitic acid impurity (%) | 1.89 | 1.13 |

It can be seen from Table 7 that the mass ratio of palmitic acid impurity was decreased by 40.2%.

Extraction and purification steps were same as the extraction and purification steps of Example 8, except that it further comprises step 5 following step 4: i.e., repeating step 4 on C16 dibasic acid secondary product to obtain C16 dibasic acid tertiary product.

The purity of the primary C16 dibasic acid product and tertiary product and the content of palmitic acid impurity were determined and calculated using the method described in Example 1 (4), as shown in Table 8 below:

**Table 8**

| C16 dibasic acid | Strain | CCTCC M2011192 | 5473 |
|---|---|---|---|
| primary product | Purity of C16 dibasic acid (%) | 82.50 | 84.70 |
| | Content of palmitic acid impurity (ppm) | 4976 | 3200 |
| Tertiary product | Purity of C16 dibasic acid (%) | 98.50 | 99.10 |
| | Content of palmitic acid impurity (ppm) | 564 | 265 |

### Example 12

The DNA fragment (SEQ ID NO: 19) in Example 9 was introduced into *Candida tropicalis* (CCTCC M 203052) by homologous recombination according to the method as described in Example 4. The positive clones were screened out according to the method as described in Example 5. The obtained single colony and the parent strain (CCTCC M 203052) were assayed for the sequence of the gene CPR-b using the same method as described in Example 6. By sequencing, it was confirmed that the sequence of the gene CPR-b in the parent strain (CCTCC M 203052) was consistent with the published sequence in GenBank with the Accession Number of AY823228, while the screened out colony carried a mutation in this gene in which the mutation was consistent with SEQ ID NO: 13. One strain was designated as 5475HYG.

The method for fermentation was according to Example 5, in which the strains used were CCTCC M 203052 and 5475HYG. After completion of fermentation, a sample of 0.5g of each fermentation broth was collected and the yield of dicarboxylic acid and the amount of lauric acid impurity were calculated, as shown in Table 9. The results indicated that the content of the lauric acid impurity in the fermentation broth by the strain 5475HYG was significantly decreased compared the parent strain CCTCC M 203052.

**Table 9**

| Strain | CCTCC M203052 | 5475HYG |
|---|---|---|
| C12 dibasic acid (mg/g) | 137.2 | 135.4 |
| Mass ratio of lauric acid impurity (%) | 1.27 | 0.62 |

From the above Examples 8-12 regarding the fermentation production of long-chain dibasic acids from different fermentation substrates, it can be seen that the content of major fatty acid impurity in the fermentation broth after fermentation was significantly decreased; compared to the parental strain, the content of fatty acid impurity could be decreased by up to around 40%, and further extraction and purification of the obtained C12, C10 and C16 dibasic acids could further reduce the impurity content, which reduces the difficulty of the later extraction and purification processes to a great extent. Moreover, as important raw materials for nylon filaments, synthetic perfumes, engineering plastics, cold-resistant plasticizers, advanced lubricating oils and polyamide hot-melt adhesives, dibasic acid products with decreased fatty acid impurity would be more favorable for the production and fabrication of downstream products, and improve the quality of downstream products.

## Claims

1. An isolated mutated CPR-b gene, homologous gene or variant thereof, relative to the GenBank Accession Number AY823228, taking the first base upstream of the start codon ATG as -1, comprising a base mutation -322G>A, and taking the first base downstream of the stop codon TAG as 1, comprising mutations 3'UTR.19C>T and 3'UTR.76_77insT; wherein the variant has at least 70% sequence identity to the mutated CPR-b gene or homologous gene thereof;
wherein preferably the sequence of the mutated CPR-b gene is set forth in SEQ ID NO: 13 or 23, or has at least 70% sequence identity thereto, more preferably, a sequence having at least about 75%, 80%, 85%, 90%, 91%, 92%, 93%, 94%, 95%, 96%, 97%, 98%, 99%, 99.18%, 99.21%, 99.25%, 99.28%, 99.32%, 99.36%, 99.39%, 99.43%, 99.46%, 99.50%, 99.53%, 99.57%, 99.61%, 99.64%, 99.68%, 99.72%, 99.75%, 99.79%, 99.82%, 99.86%, 99.89%, 99.93% or 99.96% identity thereto.

2. A microorganism containing the mutated CPR-b gene, homologous gene or variant thereof according to claim 1, which produces a long-chain dibasic acid with decreased content of a fatty acid impurity, compared to a microorganism containing a non-mutated CPR-b gene or homologous gene thereof;
wherein preferably the microorganism is selected from the group consisting of *Corynebacterium, Geotrichum candidum, Candida, Pichia, Rhodotroula, Saccharomyces* and *Yarrowia,* more preferably the microorganism is yeast, and more preferably the microorganism is *Candida tropicalis* or *Candida sake;*
wherein preferably the long-chain dibasic acid is selected from the group consisting of C9 to C22 long-chain dibasic acids, preferably C9 to C18 long-chain dibasic acids, more preferably is one or more selected from the group consisting of C10 dibasic acid, C11 dibasic acid, C12 dibasic acid, C13 dibasic acid, C14 dibasic acid, C15 dibasic acid and C16 dibasic acid;
wherein preferably the long-chain dibasic acid is at least one of C10 to C16 dibasic acids, or at least one of *n*-C10 to C16 dibasic acids, more preferably, at least one selected from the group consisting of sebacic acid, undecanedioic acid, dodecanedioic acid, tridecanedioic acid, tetradecanedioic acid, pentadecanedioic acid and hexadecanedioic acid.

3. A method of producing a long-chain dibasic acid, comprising culturing the microorganism according to claim 2, and optionally isolating, extracting and/or purifying the long-chain dibasic acid from the culture;
wherein preferably the mass ratio of a fatty acid impurity contained in the fermentation broth produced by fermentation with the microorganism is below 1.50%, and/or compared with the content of the fatty acid impurity in the long-chain dibasic acid produced by fermentation method with a conventional microorganism, such as by fermentation with a non-mutant microorganism, the fatty acid impurity in the fermentation broth is decreased by at least 5%, wherein the mass ratio is the mass percentage of the fatty acid impurity to the long-chain dibasic acid in the fermentation broth.

4. A long-chain dibasic acid with low content of a fatty acid impurity produced by a microorganism, wherein the content of the fatty acid impurity contained in the long-chain dibasic acid is more than 0, and less than 4,000 ppm, less than 1000 ppm, less than 200 ppm or less, and the fatty acid impurity comprises a saturated linear organic acid containing one terminal carboxyl group;
wherein preferably the long-chain dibasic acid is selected from C9-C22 long-chain dibasic acids, preferably C9 to C18 long-chain dibasic acids, more preferably comprises one or more selected from the group consisting of C10 dibasic acid, C11 dibasic acid, C12 dibasic acid, C13 dibasic acid, C14 dibasic acid, C15 dibasic acid and C16 dibasic acid;
wherein preferably said long-chain dibasic acid is at least one of C10 to C16 dibasic acids, or at least one of *n*-C10 to C16 dibasic acids, more preferably, at least one selected from the group consisting of sebacic acid, undecanedioic acid, dodecanedioic acid, tridecanedioic acid, tetradecanedioic acid, pentadecanedioic acid and hexadecanedioic acid.

5. The long-chain dibasic acid according to claim 4, wherein the fatty acid impurity has the formula CH₃-(CH₂)ₙ-COOH, where n≥7;
wherein preferably the fatty acid impurity comprises a long-chain fatty acid with the number of carbon atoms in the carbon chain greater than 9 and with one terminal carboxyl group;
wherein preferably the fatty acid impurity comprises any one or more of C9 fatty acid, C10 fatty acid or capric acid, C11 fatty acid, C12 fatty acid or lauric acid, C13 fatty acid, C14 fatty acid or myristic acid, C15 fatty acid, C16 fatty acid or palmitic acid, C17 fatty acid, C18 fatty acid or stearic acid, or C19 fatty acid.

6. The long-chain dibasic acid according to claim 4 or 5, wherein,
where the long-chain dibasic acid is C12 dibasic acid, the fatty acid impurity is predominantly lauric acid, and the content of the lauric acid impurity is less than 3000 ppm, preferably less than 500 ppm, 400 ppm, 300 ppm, 200ppm or less; where the long-chain dibasic acid is C10 dibasic acid, the fatty acid impurity is predominantly capric acid, and the content of the capric acid impurity is less than 2000 ppm, preferably less than 500 ppm, 400 ppm, 300 ppm, 200 ppm or less; or where the long-chain dibasic acid is C16 dibasic acid, the fatty acid impurity is predominantly palmitic acid, and the content of the palmitic acid impurity is less than 4000 ppm, preferably less than 500 ppm, 400 ppm, 300ppm or less.

7. A fermentation broth in the process of producing a long-chain dibasic acid by fermentation with a microorganism, wherein the fermentation broth contains a fatty acid impurity, and the content of the fatty acid impurity is less than 1.5%, 1.4%, 1.3%, 1.2 %, 1.1%, 1.0%, 0.9%, 0.8%, 0.7%, 0.6%, 0.5%, 0.4%, 0.3% or less, wherein the percentage is the mass percentage of the fatty acid impurity to the long-chain dibasic acid in the fermentation broth;
wherein preferably the long-chain dibasic acid is selected from C9 to C22 long-chain dibasic acids, and the fatty acid impurity comprises a saturated linear organic acid with one terminal carboxyl group;
wherein preferably the long-chain dibasic acid is selected from C9 to C18 long-chain dibasic acids, more preferably one or more selected from the group consisting of C10 dibasic acid, C11 dibasic acid, C12 dibasic acid, C13 dibasic acid, C14 dibasic acid, C15 dibasic acid and C16 dibasic acid;
wherein preferably said long-chain dibasic acid is at least one of C10 to C16 dibasic acids, or at least one of *n*-C10 to C16 dibasic acids, more preferably, at least one selected from the group consisting of sebacic acid, undecanedioic acid, dodecanedioic acid, tridecanedioic acid, tetradecanedioic acid, pentadecanedioic acid and hexadecanedioic acid;
wherein preferably the fatty acid impurity has the formula CH₃-(CH₂)ₙ-COOH, where n≥7;
wherein preferably the fatty acid impurity comprises a long-chain fatty acid with the number of carbon atoms in the carbon chain greater than 9 and with one terminal carboxyl group;
wherein preferably the fatty acid impurity comprises any one or more of C9 fatty acid, C10 fatty acid or capric acid, C11 fatty acid, C12 fatty acid or lauric acid, C13 fatty acid, C14 fatty acid or myristic acid, C15 fatty acid, C16 fatty acid or palmitic acid, C17 fatty acid, C18 fatty acid or stearic acid, or C19 fatty acid.

8. The long-chain dibasic acid according to any one of claims 4-6 or the fermentation broth according to claim 7, which is obtained or obtainable by the method according to claim 3.

9. A method of modifying a long-chain dibasic acid producing microorganism, comprising a step of directed evolution of a key gene in the pathway of the long-chain dibasic acid synthesis, wherein, compared to the microorganism before modified, the modified long chain dibasic acid producing microorganism is capable of producing the long chain dibasic acid with substantially decreased content of fatty acid impurity, wherein the key gene in the pathway of the long-chain dibasic acid synthesis is CPR-b gene and the evolved CPR-b gene is a mutated CPR-b gene, homologous gene or variant thereof according to claim 1;
wherein preferably the long-chain dibasic acid is selected from the group consisting of C9 to C22 long-chain dibasic acids, more preferably C9 to C18 long-chain dibasic acids, even more preferably one or more selected from the group consisting of C10 dibasic acid, C11 dibasic acid, C12 dibasic acid, C13 dibasic acid, C14 dibasic acid, C15 dibasic acid and C16 dibasic acid;
wherein preferably the long-chain dibasic acid is at least one of C10 to C16 dibasic acids, or at least one of *n*-C10 to C16 dibasic acids, more preferably, at least one selected from the group consisting of sebacic acid, undecanedioic acid, dodecanedioic acid, tridecanedioic acid, tetradecanedioic acid, pentadecanedioic acid and hexadecanedioic acid;
wherein preferably the fatty acid impurity comprises those with the number of carbon atoms in the carbon chain greater than 9, more preferably C10 acid (capric acid), C12 acid (lauric acid), C14 acid (myristic acid), C16 acid (palmitic acid) and/or C18 acid (stearic acid);
wherein preferably the content of the fatty acid impurity is decreased to below 300 ppm, 290ppm, 270ppm, 250ppm, 200ppm, 150ppm, 140ppm, 130ppm, 120ppm, 110ppm, 100ppm or lower.

10. The method according to claim 9, comprising steps of:
a) preparing a target gene fragment carrying a mutation by error-prone PCR;
b) preparing fragments upstream and downstream of the desired target gene necessary for homologous recombination as templates for homologous recombination with a resistance marker gene,
wherein preferably the resistance marker gene is hygromycin B;
c) preparing a complete recombinant fragment by PCR overlap extension;
d) introducing the recombinant fragment into a strain by means of homologous recombination;
e) screening a positive strain by the resistance marker;
f) screening a strain wherein the content of fatty acid impurity in the fermentation broth after the end of fermentation is significantly decreased;
g) optionally, removing the resistance marker in the screened strain by further homologous recombination.

11. A method for producing a long-chain dibasic acid, comprising:
- obtaining a long-chain dibasic acid producing microorganism containing a mutated CPR-b gene, homologous gene or variant thereof by directed evolution of the CPR-b gene, homologous gene or variant thereof in the long-chain dibasic acid synthesis pathway;
- culturing the microorganism to produce the long-chain dibasic acid by fermentation;
- optionally isolating, extracting and/or purifying the long-chain dibasic acid from the culture product;
wherein the mutated CPR-b gene, homologous gene or variant thereof, relative to GenBank Accession Number AY823228, comprises one base mutation -322G>A in its promoter region by taking the first base upstream of the start codon ATG as -1; and taking the first base downstream of the stop codon TAG as 1, comprises base mutations 3'UTR.19C>T and 3'UTR.76_77insT in its terminator region; and the variant has at least 70% sequence identity to the mutated CPR-b gene or homologous gene thereof;
wherein preferably the sequence of the mutated CPR-b gene is set forth in SEQ ID NO. 13 or 23, or has at least 70% sequence identity thereto, more preferably, a sequence having at least about 75%, 80%, 85%, 90%, 91%, 92%, 93%, 94%, 95%, 96%, 97%, 98%, 99%, 99.18%, 99.21%, 99.25%, 99.28%, 99.32%, 99.36%, 99.39%, 99.43%, 99.46%, 99.50%, 99.53%, 99.57%, 99.61%, 99.64%, 99.68%, 99.72%, 99.75%, 99.79%, 99.82%, 99.86%, 99.89%, 99.93% or 99.96% identity; wherein preferably the long-chain dibasic acid is selected from C9-C22 long-chain dibasic acids, more preferably C9-C18 long-chain dibasic acids, even more preferably one or more selected from the group consisting of C10 dibasic acid, C11 dibasic acid, C12 dibasic acid, C13 dibasic acid, C14 dibasic acid, C15 dibasic acid and C16 dibasic acid;
wherein preferably the long-chain dibasic acid is at least one of C10 to C16 dibasic acids, or at least one of *n*-C10 to C16 dibasic acids, more preferably, at least one selected from the group consisting of sebacic acid, undecanedioic acid, dodecanedioic acid, tridecanedioic acid, tetradecanedioic acid, pentadecanedioic acid and hexadecanedioic acid.

12. The method according to claim 11, wherein the microorganism is yeast, preferably *Candida tropicalis* or *Candida sake.*

13. The method according to claim 11 or 12, wherein the long-chain dibasic acid producing microorganism containing a mutated CPR-b gene, homologous gene or variant thereof is obtained or obtainable by the method according to claim 9 or 10.
